(19) Europäisches Patentamt
European Patent Office
Office européen des brevets

(11) Publication number: **0 174 717 B1**

(12) # EUROPEAN PATENT SPECIFICATION

(45) Date of publication of patent specification: **22.01.92**

(51) Int. Cl.[5]: **C07D 235/24**, C07D 235/02, C07D 413/12, C07D 417/12, C07D 403/12, C07D 401/12, A61K 31/415

(21) Application number: **85304626.6**

(22) Date of filing: **28.06.85**

The file contains technical information submitted after the application was filed and not included in this specification

(54) **Benzimidazoles, and their production formulation and use as gastric acid secretion inhibitors.**

(30) Priority: **06.07.84 GB 8417271**
**06.07.84 GB 8417272**
**02.08.84 GB 8419738**
**26.09.84 GB 8424346**
**26.09.84 GB 8424347**
**26.09.84 GB 8424350**
**26.09.84 GB 8424351**
**29.11.84 GB 8430163**
**12.04.85 GB 8509406**

(43) Date of publication of application:
**19.03.86 Bulletin 86/12**

(45) Publication of the grant of the patent:
**22.01.92 Bulletin 92/04**

(84) Designated Contracting States:
**AT DE NL SE**

(56) References cited:
**EP-A- 130 729** **EP-A- 167 943**
**EP-A- 173 664** **EP-A- 174 726**
**EP-A- 178 438** **EP-A- 0 001 279**
**EP-A- 0 005 129** **EP-A- 0 045 200**
**EP-A- 0 080 602** **DE-A- 2 548 340**
**DE-A- 3 531 487** **GB-A- 1 371 650**

(73) Proprietor: **FISONS plc**
**Fison House Princes Street**
**Ipswich Suffolk IP1 1OH(GB)**

(72) Inventor: **Cox, David**
**3 Belvoir Drive**
**Loughborough Leicestershire(GB)**
Inventor: **Ingall, Anthony Howard**
**53 Forest Road**
**Loughborough Leicestershire(GB)**
Inventor: **Suschitzky, John Louis**
**73 Kirkstone Drive**
**Loughborough Leicestershire(GB)**

(74) Representative: **Craig, Christopher Bradberry et al**
**Fisons plc 12 Derby Road**
**Loughborough Leicestershire LE11 0BB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 174 717 B1

## Description

This invention relates to new compounds, methods for their preparation and pharmaceutical formulations containing them.

A variety of benzothiazole-2-sulphinamides are known for use as vulcanisation accelerators, e.g. from US-A-2,585,155 and US-A-3,541,060, from FR-A-1,003,821 and 2,037,001, and from DE-A-1,949,615. A number of 2-(pyridylmethylsulphinyl) benzimidazoles are known for use as pharmaceuticals from EP-A-5129, EP-A-0130729 and EP-A-80602 and from GB-A-2,134,523 and a number of 2-(heterocyclicmethylsulphinyl)benzimidazoles are known from EP-A-1279, Ch-A-623,582, DE-A-2,548,340 and FR-A-2,392,021. EP-A-45,200 discloses the cytoprotective use of the compounds disclosed in EP-A-5129, CH-A-623,582, DE-A-2,548,340 and FR-A-2,392,021. EP-A-174726, EP-A-173664, EP-A-167943 and EP-A-178438 published after the priority date of this invention, disclose 2-(pyridylmethylsulphinyl) benzimidazoles. GB-A-1371650 discloses 2-(piperidinoalkyl)- and 2-(moropholinoalkyl)-sulphinyl benzimidazoles. GB-A-2141429 discloses N-alkanoyl substituted benzimidazoles.

DE-A-3531487, and its equivalents - Swedish Patent Application No 8504048.3 and NL-A-8502384 (published after the priority date of this invention) disclose 2-(phenylalkylsulphinyl) benzimidazoles.

These applications establish prior national rights in Contracting States DE, SE and NL for the following compounds:

2-(1H-2-benzimidazolylsulphinylmethyl)-N,N,4-trimethylbenzenamine,
2-(5-chloro-1H-2-benzimidazolylsulphinylmethyl)-N,N-dimethylbenzenamine,
Methyl 2-(2-dimethylaminophenylmethylsulphinyl) -1H-benzimidazole-5-carboxylate,
N,N-dimethyl-2-(5-methyl-1H-2-benzimidazolylsulphinylmethyl)benzenamine,
2-[2-(1-piperidyl)-phenylmethylsulphinyl]-1H-benzimidazole,
2-[2-(5-methoxy-1H-benzimidazolyl)sulphinylmethyl]-N,N-dimethyl-benzenamine,
2-(5-trifluoromethyl-1H-2-benzimidazolylsulphinylmethyl)-N,N-dimethyl-benzenamine,
2-(1H-2-benzimidazolylsulphinylmethyl)-4-methoxy-N,N-dimethylbenzenamine,
2-[2-(4-morpholinyl)phenylmethylsulphinyl]-1H-benzimidazole;
2-(1H-2-benzimidazolylsulphinylmethyl)benzenamine, and 2-(5-amino-1H-2-benzimidazolylsulphinylmethyl)-N,N-dimethyl-benzenamine.

According to the invention we provide compounds of formula I,

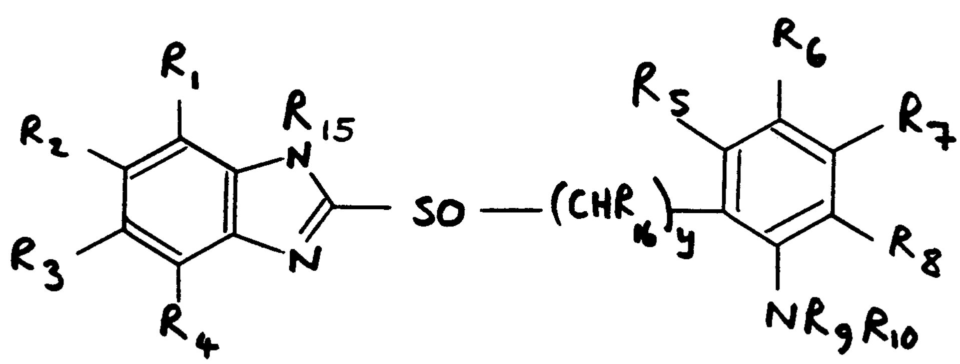

I

wherein $R_1$, $R_2$, $R_3$, $R_4$, $R_5$, $R_6$, $R_7$ and $R_8$, which may be the same or different, are each hydrogen, halogen, $C_{1-6}$-alkoxy, $C_{1-6}$-alkyl, $C_{1-6}$-fluoroalkyl, $RS(O)_n$- or $-NO_2$;

in addition, $R_2$ and $R_3$ may together form the chain -CH=CH-CH=CH-, and $R_1$, $R_2$, $R_3$ and $R_4$ may independently represent phenylcarbonyl;

$R_9$ and $R_{10}$, which may be the same or different, are each hydrogen, alkyl C1 to C6, phenyl or cycloalkyl containing up to 6 carbon atoms; or

$R_9$ and $R_{10}$ together with the nitrogen atom to which they are attached, may form a saturated or unsaturated 4 to 8 inclusive membered ring which may contain 0, 1 or 2 further hetero atoms, which ring may carry one or more substituents $R_1$, or

$R_9$ is as defined above save that it cannot form a ring with $R_{10}$, and $R_8$ and $R_{10}$, together with the nitrogen atom and the carbon atoms of the ring to which the nitrogen atom and $R_8$ are attached form a 4 to 8 inclusive membered ring which may contain 0, 1, or 2 further hetero atoms, which ring may carry one or more substituents $R_1$,

y is 0, 1 or 2;

n is 0, 1 or 2;

$R_{15}$ is hydrogen, -COOR or $C_{1-6}$-alkyl which latter is optionally substituted by -OCOR,

$R_{16}$ is H or $C_{1-6}$-alkyl;

R is hydrogen, $C_{1-6}$-alkyl, phenyl, or phenyl optionally substituted by $C_{1-6}$-alkyl;

and pharmaceutically acceptable salts thereof.

We also provide the compounds of formula I, and pharmaceutically acceptable salts thereof, for use as pharmaceuticals, in particular for use in the prevention or inhibition of gastric acid secretion.

According to the invention we also provide a process for the production of a compound of formula I, or a pharmaceutically acceptable salt thereof, which comprises

a) selective oxidation of a corresponding compound of formula VI,

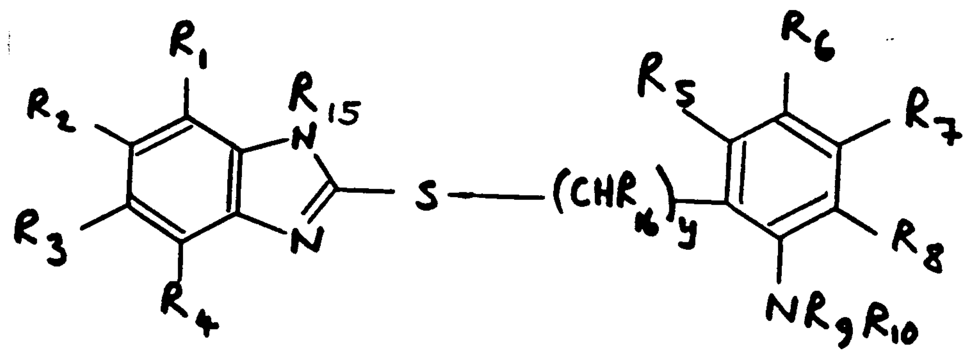

VI

in which $R_1$, $R_2$, $R_3$, $R_4$, $R_5$, $R_6$, $R_7$, $R_8$, $R_9$, $R_{10}$, $R_{15}$, $R_{16}$ and y are as defined above, or

b) production of a compound of formula I in which $R_{15}$ is as defined above save that it cannot be hydrogen, by reaction of a corresponding compound of formula I in which $R_{15}$ is hydrogen with a compound $R_{15}Z$ in which $R_{15}$ is as defined above save that it cannot be hydrogen, and Z is a good leaving group,

and where desired or necessary converting the resulting compound of formula I to a pharmaceutically acceptable salt thereof, or vice versa.

For Contracting State AT we also provide processes (a) and (b) as defined above in which $R_1$, $R_2$, $R_3$, $R_4$, $R_5$, $R_6$, $R_7$ and $R_8$ may additionally represent $-N(R)_2$ and -COOH (or an ester or amide thereof). For Contracting State AT we further provide process (c), which comprises production of a compound of formula I carrying an $-NH_2$ group by selective reduction of a corresponding compound of formula I carrying an $-NO_2$ group,

and where desired or necessary converting the resulting compound of formula I to a pharmaceutically acceptable salt thereof, or vice versa.

The oxidation of process a) may be carried out in a solvent which is inert under the reaction conditions, e.g. ethyl acetate, dichloromethane, chloroform or a mixture thereof. The reaction is preferably carried out at less than room temperature, e.g. -20° to +10°C. Suitable oxidising agents for use in the reaction are peracids, e.g. m-chloroperbenzoic acid or t-butylhydroperoxide in the presence of a suitable catalyst, e.g. vanadyl acetyl acetonate.

In process b) the good leaving group may be, for example, halogen and the reaction may be carried out in a solvent which is inert under the reaction conditions, e.g. dimethylformamide, in the presence of a base and at a temperature of from about 15° to 30°C.

In process c) the selective reduction may, for example, be carried out chemically under basic conditions, e.g. using hydrazine and Raney nickel, but is preferably carried out catalytically, e.g. using a $PtO_2$ catalyst and ethanol as the reaction medium.

The compounds of formula VI may be made by conventional processes known *per se*, e.g. by reaction of a compounds of formula VII,

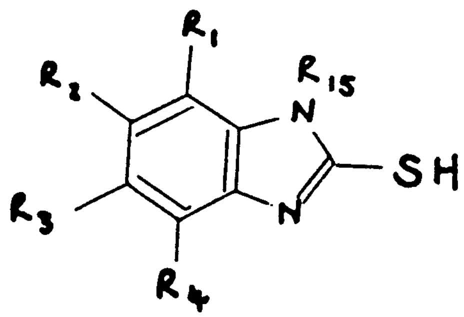

VII

in which $R_1$, $R_2$, $R_3$, $R_4$ and $NR_{15}$ are as defined above,
with a compound of formula VIII,

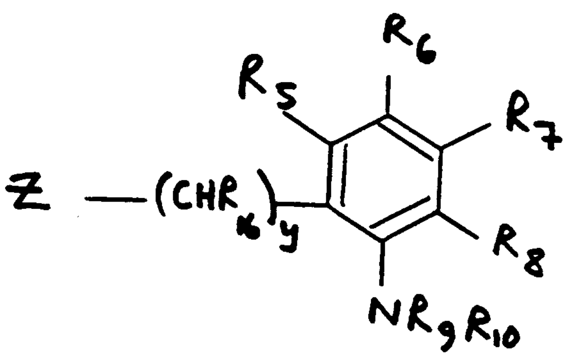

VIII

in which $R_5$, $R_6$, $R_7$, $R_8$, $R_9$, $R_{10}$, $R_{16}$ and y are as defined above, and
Z is a good leaving group, e.g. halogen (chlorine).

The reaction may be carried out in a suitable solvent, eg N,N-dimethylformamide, and in the presence of an acid acceptor, eg potassium carbonate.

The compounds of formulae VII and VIII are either known or may be made from known compounds using conventional techniques known per se. The production of the starting materials for the above reactions is more fully described in GB-A-: 8509406 from which the present case draws priority.

The compounds of formula I, and the intermediates therefor, may be isolated from their reaction mixtures using conventional techniques.

Pharmaceutically acceptable salts of the compounds of formula I include salts with suitable organic or inorganic acids, e.g. with a hydrohalic, sulphuric, alkanesulphonic, tartaric or citric acid. We also provide, when the compound of formula I carries a -COOH, or other acidic, group, salts with suitable organic or inorganic bases, e.g. ammonium, alkali metal, alkaline earth metal, alkylamino salts. The benzimidazole nucleus itself is acidic and can form salts with appropriate bases as above.

The compounds of formula I, and pharmaceutically acceptable salts thereof, are useful because they possess pharmacological activity in animals; in particular they are useful because they prevent or inhibit gastric acid secretion, e.g. in the test set out in Am.J.Physiol., 1982, 243(6), 505-510. The compounds of formula I are also useful as intermediates in the synthesis of other chemicals.

The new compounds are thus indicated for use in the prevention or inhibition of gastric acid secretion, and/or the treatment of conditions normally involving excess gastric acid secretion, e.g. peptic, duodenal, gastric, recurrent or stormal ulceration, dyspepsia, duodenitis, Zollinger-Ellison syndrome, reflux oesophagitis and the management of haemorrhage, e.g. from erosion of ulcers in the upper gastrointestinal tract, especially when a major blood vessel is not involved. The compounds may also be used to treat gastritis or dyspepsia associated with administration of non-steroidal anti-inflammatory drugs, in the prophylaxis of gastrointestinal haemorrhage from stress ulceration in seriously ill or burned patients, in the prophylaxis of recurrent haemorrhage in patients with bleeding peptic ulcers, before general anaesthesia in patients at risk of acid aspiration syndrome (Mendelson's syndrome) and to reduce the chance of haemorrhage in patients with leukaemia, graft versus host disease or with severe hepatic failure. The above conditions may be treated whether or not they are associated with excess gastric acid secretion.

Patterns of therapeutic use which may be mentioned are:-

a) a high dose initially, for say 2-4 weeks, followed by lower-dose maintenance therapy after the condition has improved, e.g. the ulcer has healed,

b) as in a) above, but the maintenance therapy including a cytoprotective agent, e.g. a $PGE_2$ derivative,

c) combination therapy, using a low dose of the compound of the invention in association with a low, well-tolerated dose of a cytoprotectant and/or antacid,

d) intermittent dosing, e.g. every second day, may be appropriate as maintenance therapy.

For the above mentioned uses the dosage administered will, of course, vary with the compound employed, the mode of administration and the treatment desired. However, in general, satisfactory results are obtained when the compounds are administered at a dosage of from $10^{-6}$M to $10^{-4}$M in the test set out in Am.J.Physiol, 1982, 243 (6), 505-510. For man the indicated total daily dosage is in the range of from 1mg to 3,000mg, preferably 5 to 500mg, and more preferably from 10mg to 200mg, which may be administered in divided doses from 1 to 6 times a day or in sustained release form. Thus unit dosage forms suitable for administration comprise from about 1.0mg to 600mg of the compound admixed with a solid or liquid pharmaceutically acceptable diluent, carrier or adjuvant.

The compounds of formula I, and pharmaceutically acceptable salts thereof, have the advantage that they are more readily absorbed, or are less irritant to the GI tract, or have less toxic side effects, or are more active, or are more stable to gastric acid when administered by ingestion than compounds of similar structure.

We prefer at least one of $R_1$, $R_2$, $R_3$ and $R_4$, and at least one of $R_5$, $R_6$, $R_7$ and $R_8$ to be other than hydrogen. When $R_1$, $R_2$, $R_3$, $R_4$, $R_5$, $R_6$, $R_7$ or $R_8$ is halogen it may be chlorine or fluorine.

We particularly prefer each of $R_9$ and $R_{10}$ to contain 1 or 2 carbon atoms.

When $R_9$ and $R_{10}$ together with the nitrogen atom to which they are attached form a ring, the ring may contain a further nitrogen, oxygen and/or sulphur atom. We prefer that ring to be a piperidino or morpholino ring.

When any of $R_1$ to $R_8$ represent an ester we prefer it to be with a $C_{1-6}$-alcohol, e.g. with an alkanol. When any of $R_1$ to $R_8$ represent an amide they may be, for example, an unsubstituted or a mono- or di-alkyl substituted amide.

Specific groups $R_1$ to $R_4$ include hydrogen, methyl, chloro, methoxy, $CF_3$, $NO_2$, p-toluenesulphonyl, and additionally $-NH_2$ and methoxycarbonyl for the Contracting State AT, or $R_2$ and $R_3$ may together form the chain $-CH=CH-CH=CH-$.

We prefer y to be 0 or 1. We also prefer $R_{16}$ to be selected from H and methyl, most preferably $R_{16}$ represents H.

Specific groups $R_5$ to $R_8$ include hydrogen, methyl, chloro, propyl, methoxyl and butyl.

When $R_8$ and $R_{10}$, together with the nitrogen atom and the carbon atoms of the ring to which they are attached, form a ring, we prefer that ring to be a piperidino ring, e.g. an N-methyl piperidino ring.

Specific groups $R_{15}$ are $-CH_2OCO-(^t butyl)$, $-CO_2Et$ and methyl.

According to the invention we provide the following specific group of compounds of formula I,

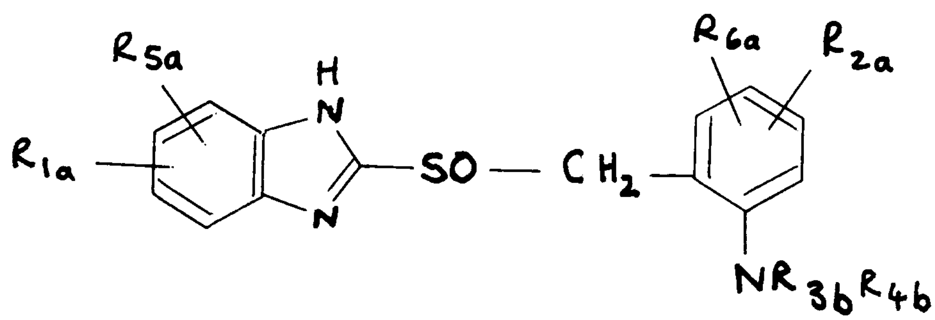

Ib

in which $R_{1a}$, $R_{2a}$, $R_{5a}$ and $R_{6a}$, which may be the same or different, are each hydrogen, halogen, $C_{1-6}$-alkoxy or $C_{1-6}$-alkyl, and

$R_{3b}$ and $R_{4b}$, which may be the same or different, are each hydrogen or $C_{1-6}$-alkyl.

Certain of the compounds of formula VI are novel and the invention also provides these novel compounds. Of particular interest are compounds of formula VI in which y is more than 0.

According to our invention we also provide a pharmaceutical composition comprising (preferably a minor proportion of) a compound of formula I, or a pharmaceutically acceptable salt thereof, as active

ingredient, in admixture with a pharmaceutically acceptable adjuvant, diluent or carrier. Examples of suitable adjuvants, diluents or carriers are:- for tablets and dragees; lactose, starch, talc or stearic acid; for capsules, tartaric acid or lactose; for suppositories, natural or hardened oils or wax; and for injections (i.m. or i.v.) or enemas water, surfactants and preservatives. The compounds may also be administered transdermally, e.g. in an ointment base. The compound of formula I, or the pharmaceutically acceptable salt thereof, preferably has a mass median diameter of from 0.01 to 10 microns. The compound of such particle size may be made by grinding or milling followed if necessary by particle size classification using, for example, a sieve. The compositions may also contain suitable preserving, stabilising, and wetting agents, solubilizers, sweetening and colouring agents and flavourings. The compositions may, if desired, be formulated in sustained release form.

The compounds may, if desired, be co-administered, with (e.g. as a mixture with) an antacid buffer.

We prefer compositions which are designed to be taken by ingestion or rectally and to release their contents in the intestine. We particularly prefer compositions which will pass through the acidic parts of the gastrointestinal tract unaffected, e.g. enteric coated formulations.

The compounds of formula I are optically active and may be resolved into their optical isomers using conventional techniques known per se. The invention therefore provides the compounds as their optical isomers, or as mixtures, e.g. racemic mixtures, thereof.

The invention is illustrated, by the following Examples in which temperatures are in degrees centigrade.

Example 1

N,N-Dimethyl 2-(1H-benzimidazol-2-ylsulphinylmethyl)benzenamine

a) N,N-Dimethyl 2-(1H-benzimidazol-2ylthiomethyl)benzenamine

2-Dimethylaminobenzyl chloride hydrochloride (8.18g) was dissolved in dry dimethylformamide (100ml), treated with 2-mercaptobenzimidazole (5.4g) and anhydrous potassium carbonate (11.0g) and the resulting mixture stirred at room temperature overnight. The reaction mixture was poured into water and extracted with ethyl acetate which was washed with water and dried over magnesium sulphate. The solvent was evaporated and the residue recrystallised from toluene to give 5.68g of a cream coloured solid. The product was eluted down a flash chromatography column with dichloromethane/ethyl acetate (9:1) as eluant to give a colourless solid mp 158-160°.

Elemental Analysis:

Found: C, 68.05, H, 6.09, N, 15.1, S, 11.34.

$C_{16}H_{17}N_3S$

Required: C, 67.8, H, 6.01, N, 14.85, S, 11.31%

b) N,N-Dimethyl 2-(1H-benzimidazol-2ylsulphinylmethyl)benzenamine

98% m-Chloroperbenzoic acid (0.67g) was added portionwise over a few minutes to a stirred solution of the product of step a) (1.0g) in dichloromethane (30ml) at 0°. The reaction mixture was stirred for 0.5h, washed with aqueous saturated sodium bicarbonate solution then brine and dried. The solvent was evaporated and the residue eluted down a flash chromatography column using dichloromethane/ethyl acetate (7:3) as eluant to give 0.6g of colourless solid mp 120-121°.

Example 2

By the method described in Example 1, and using the appropriate starting materials, may be prepared the following compounds:

a)

i) N,N-Dimethyl-2-(5,6-dimethyl-1H-2-benzimidazolylthiomethyl)benzenamine. mp 144-146°.

ii) N,N-Dimethyl-2-(5,6-dimethyl-1H-2-benzimidazolyl sulphinylmethyl)benzenamine, mp 141-142°;

206-207°.

b)

i) 2-(1H-2-Benzimidazolylthiomethyl)-N,N,4-trimethyl -benzenamine, mp 159-161°.

ii) 2-(1H-2-Benzimidazolylsulphinylmethyl)-N,N,4-trimethyl-benzenamine, mp 133-134°.

c)

i) 2-(1H-2-Benzimidazolylthiomethyl)-4-chloro-N,N-dimethyl-benzenamine, mp 148-151°.

ii) 2-(1H-2-Benzimidazolylsulphinylmethyl)-4-chloro -N,N-dimethyl-benzenamine, mp 148-151°.

d)

i) 2-(5-Chloro-1H-2-benzimidazolylthiomethyl)-N,N-dimethyl-benzenamine, mp 49-52°.

ii) 2-(5-Chloro-1H-2-benzimidazolylsulphinylmethyl)-N,N-dimethyl-benzenamine, mp 121-123°.

e)

i) 2-(5,6-Dichloro-1H-2-benzimidazolylthiomethyl)-N,N-dimethylbenzenamine, mp. 128-130°.

ii) 2-(5,6-Dichloro-1H-2-benzimidazolylsulphinyl methyl)-N,N-dimethyl-benzenamine, mp. 147-149°.

f)

i) Methyl 2-(2-dimethylaminophenylmethylthio)-1H-benzimidazole-5-carboxylate, mp 127-129°.

ii) Methyl 2-(2-dimethylaminophenylmethylsulphinyl)-1H-benzimidazole-5-carboxylate, mp 130° - (decomp).

g)

i) N,N-Dimethyl-2-(5-methyl-1H-2-benzimidazolyl thiomethyl)benzenamine, mp 141-143°.

ii) N,N-Dimethyl-2-(5-methyl-1H-2-benzimidazolyl sulphinylmethyl)benzenamine, mp 50-52°.

h)

i) 2-[2-(1-Piperidyl)-phenylmethylthio]-1H-benzimidazole, mp 171-172°.

ii) 2-[2-(1-Piperidyl)-phenylmethylsulphinyl]-1H-benzimidazole, mp 160-161°.

i)

i) 2-(1H-2-Benzimidazolylthiomethyl)-N,N-diethyl-benzenamine, mp 127-128°.

ii) 2-(1H-2-Benzimidazolylsulphinylmethyl)-N,N-diethyl-benzenamine, mp 109°.

j)

i) 2-[2-(5-Methoxy-1H-benzimidazolyl)thiomethyl]-N,N -dimethyl-benzenamine. MS: $M^+$ 329.

ii) 2-[2-(5-Methoxy-1H-benzimidazolyl)sulphinyl methyl]-N,N-dimethyl-benzenamine.

Found: C60.36% H5.81% N11.89% S9.69%

$C_{17}H_{19}N_3O_2S.{}^1/7\ CH_2Cl_2$

reqs: C60.30% H5.65% N12.30% S9.38%

k)

i) 2-(5-Trifluoromethyl-1H-2-benzimidazolylthio methyl)-N,N-dimethyl-benzenamine, mp 50-51°.

ii) 2-(5-Trifluoromethyl-1H-2-benzimidazolyl sulphinylmethyl)-N,N-dimethyl-benzenamine, mp 50-51°.

l)

i) N,N-Dimethyl-2-(5-nitro-1H-2-benzimidazolyl thiomethyl)-benzenamine, mp 146-148°.

ii) N,N-Dimethyl-2-(5-nitro-1H-2-benzimidazolyl sulphinylmethyl)-benzenamine, mp 105-106° (d).

m)

i) [2-(2-N,N-Dimethylaminophenylmethylthio)-1H-5-benzimidazolyl]phenyl methanone, mp 62°.

ii) [2-(2-N,N-Dimethylaminophenylmethylsulphinyl) -1H-5-benzimidazolyl]phenyl methanone, mp 74°.

n)

i) 2-(5,6-Dimethoxy-1H-2-benzimidazolylthiomethyl) -N,N-dimethyl-benzenamine, mp 93-95°.

ii) 2-(5,6-Dimethoxy-1H-2-benzimidazolylsulphinyl methyl)-N,N-dimethyl-benzenamine, mp 142-144°.

o)

i) N,N-Dimethyl-2-(4-trifluoromethyl-1H-2-benzimidazolylthiomethyl)-benzenamine, mp 93-95°.

ii) N,N-Dimethyl-2-(4-trifluoromethyl-1H-2-benzimidazolylsulphinylmethyl)-benzenamine, mp 129-130°.

p)

i) N,N-Dimethyl-2-(1H-2-naphtho[2,3-d]imidazolyl thiomethyl)-benzenamine, mp 178°(d).

ii) N,N-Dimethyl-2-(1H-2-naphtho[2,3-d]imidazolyl sulphinylmethyl)-benzenamine, mp 129°(d).

q)

2-(2-(1H-Benzimidazolyl)sulphinyl)benzenamine, mp 202-203° shrinks at 160°.

Example 3

2-(1H-2-Benzimidazolylsulphinylmethyl)-N,N-dimethyl-6-propyl-benzenamine

a) 2-Methoxy-3-propylbenzoic acid

Methyl 2-methoxy-3-propylbenzoate ( 1.9g) was dissolved in methanol (300ml). Sodium hydroxide (16.6g) in water (100ml) was added and the mixture was heated at reflux for three hours. The solvent was evaporated and the mixture acidified with dilute hydrochloric acid. The product was extracted with ethyl acetate (800ml), washed with water, dried over magnesium sulphate and the solvent evaporated. Extraction of the resulting brown oil with hot pentane yielded 25.9g of the sub-title compound as a yellow solid, mp 55-58°.

b) N-(1,1-Dimethyl-2-hydroxyethyl)-2-methoxy-3-propyl benzamide

2-Methoxy-3-propylbenzoic acid (25.3g) in dry dichloromethane (400ml) was heated at reflux temperature with thionyl chloride (17ml) for three hours and then stirred at room temperature for 14 hours. The solvent was evaporated and the product distilled using a Kugelruhr apparatus (air bath temperature 135°; 0.35mmHg) to yield 24.1g of a pale yellow oil. This oil was dissolved in dry dichloromethane (200ml) and added gradually to a stirred solution of 2-amino-2-methyl propanol (20.2g) in dichloromethane (200ml) at 0° under $N_2$. The reaction mixture was stirred at room temperature for 18 hours. The product was extracted with chloroform (300ml) and washed with dilute hydrochloric acid (150ml), sodium bicarbonate solution (150ml) and brine (100ml) and then dried over magnesium sulphate. After evaporation of the solvent the sub-title compound was crystallised from cyclohexane as a white solid (20.4g), mp 95-96.5°.

c) 4,5-Dihydro-2-[2-methoxy-3-propylphenyl]-4,4-dimethyloxazole

The product from step b) (20.4g) was stirred in dry dichloromethane (200ml) and cooled to 0°. Thionyl chloride (17ml) was added and the reaction mixture stirred at room temperature for two hours. The solvent and thionyl chloride were evaporated and the residue was treated with ether. Water was added to the solid and the mixture was basified with dilute sodium hydroxide solution. The product was extracted with ether (500ml), washed with brine (150ml) and dried over magnesium sulphate. The solvent was then evaporated and the product purified by flash chromatography, using 10% ethyl acetate/90% petroleum ether as eluant, and by distillation using a Kugelruhr apparatus (air bath temperature 135°; 0.7mmHg) to yield the sub-title compound (17g) as a colourless oil.

d) 2-(4,5-Dihydro-4,H-dimethyl-oxazol-2-yl)-N,N-dimethyl-6-propyl-benzenamine

Dimethylamine (9ml) was added to dry tetrahydrofuran (120ml) and the mixture cooled to -15° and stirred under $N_2$ during the addition of n-butyl lithium solution (81ml of 1.6M in hexane). The reaction mixture was stirred at -16° for 40 minutes. The product from step c) (16g) in dry tetrahydrofuran (100ml) was added and the mixture allowed to warm to room temperature and then stirred for 20 hours. The reaction mixture was quenched with water and the product extracted with ethyl acetate (500ml), washed with brine (100ml) and dried over magnesium sulphate. The solvent was evaporated and the product distilled using a Kugelruhr apparatus (air bath temperature 135°; 0.25mmHg) to yield 16.4g of the sub-title compound as a pale yellow oil.

e) 2-Dimethylamino-3-propylbenzenemethanol

The product from step d) (17.3g) was heated at reflux in 2M dilute hydrochloric acid (480ml) for 20 hours. The solvent was evaporated and the residue dried over phosphorous pentoxide. This product was then dissolved in dry tetrahydrofuran (500ml), cooled in ice and stirred under $N_2$ during the addition of borane-tetrahydrofuran (300ml of 1M in tetrahydrofuran). The reaction mixture was stirred at room temperature for 68 hours and was then quenched with methanol. The solvent was evaporated and the product was extracted with ethyl acetate, washed with sodium bicarbonate solution (150ml) and with brine (150ml) and dried over magnesium sulphate. The solvent was evaporated and the product distilled using a Kugelruhr apparatus (air bath temperature 156°; 1.0mmHg) to yield the sub-title compound (13.2g) as a pale yellow oil.

f) 2-Chloromethyl-6-propyl-N,N-dimethylbenzenamine hydrochloride

The product from step e) (13.1g) was cooled to 0° in dry dichloromethane (50ml) and stirred during the addition of thionyl chloride (6ml). The mixture was heated at reflux for 1.5 hours. The solvent was evaporated and ethereal HCl added. The product was collected and then triturated with dry ether to yield 6.8g of the sub-title compound as a cream solid. Mass spectrum:- m/e 211/213.

g) 2-(1H-2-Benzimidazolylthiomethyl)-N,N-dimethyl-6-propyl-benzenamine

The product of step f) was converted to the sub-title compound (mp 147-150°) by the method of Example 1a.

h) 2-(1H-2-Benzimidazolylsulphinylmethyl)-N,N-dimethyl-6-propyl benzenamine

The product of step g) was converted to the title compound (mp 145-147.5°) by the method of Example 1b.

Example 4

By the method described in Example 3, and using the appropriate starting materials, may be prepared the following compounds:

a)
- i) 2-(1H-2-Benzimidazolylthiomethyl)-4-methoxy -N,N-dimethyl-benzenamine, mp 144-145°.
- ii) 2-(1H-2-Benzimidazolylsulphinylmethyl)-4-methoxy -N,N-dimethyl-benzenamine, mp 130-131°.

b)
- i) 2-(1H-2-Benzimidazolylthiomethyl)-N-ethyl-N-propyl-benzenamine, mp 121°.
- ii) 2-(1H-2-Benzimidazolylsulphinylmethyl)-N-ethyl-N-propyl-benzenamine, mp 114°.

c)
- i) 2-[2-(4-Morpholinyl)phenylmethylthio]-1H-benzimidazole, mp 170°.
- ii) 2-[2-(4-Morpholinyl)phenylmethylsulphinyl]-1H-benzimidazole, mp 74-76°.

Example 5

2-(1,2,3,4-Tetrahydro-1,6-dimethylquinolin-8-ylmethylsulphinyl)-1H-benzimidazole

a) 1,2,3,4-Tetrahydro-6-methylquinoline

6-Methylquinoline (5.16g; 36mmole) and pyridine/borane complex (13.2ml; 144mmole) in acetic acid (75ml) were stirred at room temperature for 18 hours. The product mixture was treated with dilute aqueous HCl (30ml) with stirring and then basified (40% NaOH, then $NaHCO_3$ to pH 8) and extracted with ethyl acetate (3x). The combined organics were washed with water (3x), dried ($Na_2SO_4$) and evaporated to yield a brown oil which was flash-chromatographed. Petroleum ether (bp 40-60°)/ether (3/1) yielded the sub-title compound as a low melting solid (4.7g 67%). m/e 147 (base peak).

b) 1,2,3,4-Tetrahydro-1,6-dimethylquinoline

6-Methyltetrahydroquinoline (3.8g; 25.8mmole) in dry methylene chloride (75ml) was treated with trimethyloxonium fluoroborate (5.2g; 3.5mmole) and stirred at room temperature for 20 hours. The mixture was poured into saturated aqueous sodium hydrogen carbonate and the organic layer run off. The aqueous layer was extracted with $CHCl_3$ (2x). The combined organics were washed with water (2x), dried ($Na_2SO_4$) and evaporated to give a yellow oil which was flash-chromatographed. Petroleum ether (bp 40-60°)/ether (5/1) eluted the sub-titled compound as a pale yellow oil. m/z 161 (MW = base peak), 160, 146, 145, 144, 131, 117, 91, 77.

c) 1,2,3,4-Tetrahydro-1,6-dimethylquinoline-8-carboxaldehyde

Phosphorylchloride (1.17ml; 1.982g; 12.5mmole) was added dropwise to a solution of the product of step b) (2.1g; 10.3mmole) in dry dimethylformamide (7ml) under $N_2$ in an ice-bath with stirring. The reaction

mixture was heated to 120° (momentarily) and then held at 80° for 2 hours. The mixture was cooled, poured into dilute aqueous $NaHCO_3$ and extracted with ethyl acetate (3x). The combined organics were washed with water (3x), dried ($Na_2SO_4$) and evaporated to yield the sub-title compound as a yellow oil 960mg (49%).

m/z 189 (m.w. = base peak), 172, 160, 144, 132, 117, 105, 91. $^1$H NMR ($CDCl_3$) aldehyde at $\delta$ 10.06.

d) 1,2,3,4-Tetrahydro-8-hydroxymethyl-1,6-dimethyl-quinoline

Sodium borohydride (300mg; 7.94mmole) was added portionwise to the product of step c) (1.5g; 7.94mmole) in ethanol with stirring at room temperature over 10 minutes. The mixture was stirred for a further 20 minutes, poured into water, and extracted with ethyl acetate (3x). The combined organics were washed with water (2x), dried ($Na_2SO_4$) and evaporated to give the sub-title compound as a viscous pale yellow oil 1.41g (93%).

m/z (mono TMS derivative) 263 (MW), 248 (base peak), 172, 73.

e) 1,2,3,4-Tetrahydro-8-chloromethyl-1,6-dimethyl-quinoline hydrochloride

The product of step d) (1.4g; 7.33mmole) in dry benzene (10ml) was treated portionwise with thionyl chloride (0.8ml; 1.31g; 11mmole) in a cold water-bath with stirring. The mixture was allowed to warm to room temperature (2 hours) and then heated at 50° (1 hour). It was then cooled again and treated with ethereal HCl (2ml) and evaporated to dryness. The resulting brown solid was triturated with ether and filtered off to yield the sub-title compound as a light brown solid 1.73g (96%).

m/z 209/11 (M.W.), 174 (base peak), 158, 145, 131, 119, 91.

f) 2-(1,2,3,4-Tetrahydro-1,6-dimethyl-quinolin-8-ylmethylthio)-1H-benzimidazole

The product of step e) was converted to the sub-title compound (mp 85-88°C) by the method of Example 1a).

g) 2-(1,2,3,4-Tetrahydro-1,6-dimethylquinolin-8-ylmethylsulphinyl)-1H-benzimidazole

The product of step f) was converted to the title compound (mp 112-113°) by the method of Example 1b).

Example 6

By the method described in Example 5, and using the appropriate starting materials, may be prepared the following compounds:

a)
- i) 2-(1H-2-Benzimidazolylthiomethyl)-N,N,3,4,5-pentamethyl-benzenamine, mp 161.5-162.5°.
- ii) 2-(1H-2-Benzimidazolylsulphinylmethyl)-N,N,3,4, 5-pentamethyl-benzenamine, mp 122-123°.

b)
- i) 2-(1H-2-Benzimidazolylthiomethyl)-4-methoxy-N,N,3,5-tetramethyl-benzenamine, mp 157-158°.
- ii) 2-(1H-2-Benzimidazolylsulphinylmethyl)-4-methoxy-N,N,3,5-tetramethyl-benzenamine, mp 138-139°.

c)
- i) 2-(1H-2-Benzimidazolylthiomethyl)-N,N-dimethyl-4-(1,1-dimethylethyl)-benzenamine. mp 166-167°.
- ii) 2-(1H-2-Benzimidazolylsulphinylmethyl)-N,N-dimeth yl-4-(1,1-dimethylethyl)-benzenamine, mp 130°.

Example 7

2-[1-(2-Dimethylaminophenyl)ethylsulphinyl]-1H-benzimidazole

a) 1-(2-Dimethylaminophenyl)-ethanol

A Grignard reagent was prepared from 2-bromo-N,N-dimethylaniline (10.0g) and magnesium (1.4g) in dry ether (60mls) with iodine (1 crystal). The reagent was cooled to 0° and stirred under a nitrogen atmosphere. A solution of acetaldehyde (3.34mls) in dry ether (20 mls) was added dropwise over 30 mins. After stirring at 0° for 1 hour the mixture was allowed to warm to room temperature. After a further 2 hours

an aqueous solution of ammonium acetate was added. After 10 mins the layers were allowed to separate. The aqueous layer was extracted with ether and the combined ether extracts were washed with water and brine and then dried and evaporated to leave a dark yellow oil 7.5g. Flash chromatography (1:1 ether/petroleum ether) produced the required product as a clear yellow oil 3.7g.
NMR ($CDCl_3$) $\delta$ 7.2m(4H) 6.8broad (1H) 5.12q(1H) 2.73S(6H) 1.55d(3H)

b) 2-[1-(2-Dimethylaminophenyl)-ethylthio]-1H-benzimidazole

A solution of 2-(2-dimethylaminophenyl)-ethanol (3.6g) in dry benzene (50mls) was cooled in an ice bath and thionyl chloride (1.75mls) was added dropwise. After stirring for 1 hour the mixture was warmed to room temperature and stirring continued for 2 hours. The mixture was concentrated in vacuo and azeotroped with benzene. The residue was taken up in dry dimethylformamide (50mls) and stirred. To this solution was added a solution of 2-mercaptobenzimidazole (3.22g) in dry dimethylformamide (30mls), followed by potassium carbonate (7.5g). The mixture was stirred at room temperature for 18 hours and then poured onto water containing brine, and extracted with ethyl acetate. The combined extracts were washed with water and brine and then dried and evaporated to leave a pale brown solid 6.1g. Flash chromatography produced the product as a buff solid 3.4g.
NMR ($CDCl_3$) $\delta$ 7.0-7.7m(8H) 5.22q(1H) 2.95S(6H) 1.80d(3H)

c) 2-[1-(2-Dimethylaminophenyl)-ethylsulphinyl]-1H-benzimidazole

A solution of the product of step b) (3g) in ethyl acetate (350mls) was cooled to -20° and a solution of metachloroperbenzoic acid (1.83g) in ethylacetate (50mls) was added. After stirring for 1 hour the mixture was concentrated in vacuo. The resulting gum was dissolved in a minimum of dichloromethane and placed on a flash chromatography column. Elution with 1:1 ether/petroleum ether produced recovered starting material 1.5g plus both diastereomers of the title compound: Least polar diastereomer 437mg, mp 119-120°. Most polar diastereomer 298mg, mp 103-105°.

Example 8

[2-(2-Dimethylaminophenylmethylsulphinyl)-1H-benzimidazol-1-yl]methyl-2,2-dimethylpropanoate

A solution of 2-(1H-2-benzimidazolylsulphinylmethyl)-N,N-dimethylbenzenamine (1.5g, 5mM) and chloromethylpivalate (1ml, 6.9mM) in dry dimethylformamide (20ml) containing anhydrous potassium carbonate (1.4g, 10,0mM) was stirred at 25° for 16 hours. The mixture was quenched with water (50ml) and extracted with ethyl acetate (3x100ml). The organic phase was washed with brine (2x25ml), dried over magnesium sulphate, filtered and evaporated to leave a yellow oil which was purified by flash chromatography eluting with dichloromethane/ethyl acetate (5:1). The required fractions were evaporated to leave a yellow oil which solidified on standing. The solid was triturated with pentane, filtered and dried under vacuum (1.2g); mp 70-71°.

Similarly prepared were:

1. Ethyl 2-(2-dimethylaminophenylmethylsulphinyl)-1H-1-benzimidazole carboxylate hemihydrate, mp 75-77°.
2. 2-[1-Methyl-(1H-2-benzimidazolylsulphinylmethyl)]-N,N-dimethylbenzenamine, ms: m/e 313.

Example 9

N,N-Dimethyl-2-[5-(4-methylphenylsulphonyl)-1H-2-benzimidazolylsulphinylmethyl] benzenamine

a) 5-(4-Methylphenylsulphonyl)-1H-benzimidazole-2(3H)-thione

4-Toluenesulphonylbenzene-1,2-diamine (2.6g) was dissolved in dimethylformamide (50ml) and treated at 60° with carbon disulphide (6ml) under nitrogen for 18 hours. The cooled solution was poured into ice-water to afford a yellow precipitate of the subtitle compound, mp 200°.

b) N,N-Dimethyl-2-[5-(4-methylphenylsulphonyl)-1H-2-benzimidazolylthiomethyl]benzenamine

The product of step a) was converted to the sub-title compound (mp 81°) by the method of Example

1a).

c) N,N-Dimethyl-2-[5-(4-methylphenylsulphonyl)-1H-2-benzimidazolylsulphinylmethyl] benzenamine

The product of step b) was converted to the title compound (mp 85°) by the method of Example 1b).

Example 10

By a similar method to that of Example 9 the following compounds were prepared:

a)

i) 2-(4,7-Dimethoxy-1H-2-benzimidazolylthiomethyl)-N,N-dimethyl-benzenamine, mp 142-144°.
ii) 2-(4,7-Dimethoxy-1H-2-benzimidazolylsulphinyl methyl)-N,N-dimethyl-benzenamine, mp 61°.

Example 11

2-(1H-2-Benzimidazolylsulphinylmethyl)benzenamine

a) N-(2-Hydroxymethylphenyl)-2,4,6-trimethylbenzene sulphonamide

A solution of (2-([(2,4,6-trimethylphenyl)sulphonyl] amine)benzoic acid (5.0g) in dry tetrahydrofuran (80ml) was stirred in an ice bath under nitrogen and treated with diborane-tetrahydrofuran complex (17.3ml, of a 1 molar solution). The reaction mixture was stirred for 3 hours at room temperature cooled to 0° and more diboran-tetrahydrofuran complex (17.3ml, 1 molar solution) added and stirring continued at room temperature overnight. The reaction was again cooled to 0°, more diboran-tetrahydrofuran complex (17.3ml, 1 molar solution) added and stirring continued at room temperature for 3 hours. Dilute hydrochloric acid was added continuously and the mixture diluted with water and extracted with ethyl acetate which was washed with water and dried over magnesium sulphate. The solvent was evaporated to give 4.0g of the required product as an oil. The structure was confirmed by nmr and ms.

b) N-[2-Chloromethylphenyl)-2,4,6-trimethylbenzenesulphonamide

The product of step a) (4.0g) in dry dichloroethane (80ml) was treated with thionyl chloride (1.15ml) at room temperature with stirring. The reaction mixture was stirred for 5 hours, more thionyl chloride (0.1ml) was added and stirring continued overnight. The reaction mixture was then poured into water, and the organic layer separated. The aqueous layer was washed with dichloromethane and the organic solutions combined, dried over magnesium sulphate and the solvent evaporated to give 4.06g of the sub-title compound as a pale yellow oil.

c) N-[2-(1H-2-Benzimidazolylthiomethyl)phenyl]-2,4,6 -trimethylphenylsulphonamide

The product of step b) (4.06g) and 1,3-dihydro-2H-benzimidazole-2-thione (1.9g) were stirred with anhydrous potassium carbonate (2.1g) in dry dimethylformamide (70ml) for 3 hours. The reaction mixture was poured into water and the precipitated product collected by filtration, washed well with water and dried to give 4.39g of the required product as a buff coloured powder, mp 202-203°.

d) 2-(1H-2-Benzimidazolylthiomethyl)benzenamine

The product of step c) (3.87g) and anisole (4.83ml) were treated at room temperature with methanesulphonic acid (29ml) with stirring. The deep red reaction mixture was stirred for 27 hours, poured slowly into an excess of aqueous sodium bicarbonate solution and extracted with ethyl acetate, which was then washed with brine and dried. The solvent was evaporated and the residue eluted down a flash chromatography column using dichloromethane/ethyl acetate (4:1) as eluant to give 1.43g of the required product as a light brown solid. mp 270° (melts at 139° and resolidifies).

e) 2-(1H-2-Benzimidazolylsulphinylmethyl)benzenamine

The product of step d) was oxidised in the same manner as in Example 1b, to give, after recrystallisation from ethanol, the title compound as a fluffy colourless solid. mp 177°(d).

Example 12

2-(5-Amino-1H-2-benzimidazolylsulphinylmethyl)-N,N-dimethyl -benzenamine

N,N-Dimethyl-2-(5-nitro-1H-2-benzimidazolylsulphinylmet hyl)benzenamine (2.2g) was hydrogenated in ethanol (150ml) containing $PtO_2$(0.4g) under 1 atmosphere pressure for 24 hours. The catalyst was removed, and solvent evaporated in vacuo. The residue was chromatographed ($SiO_2$/1:10 methanol-ethyl acetate) to afford the title compound. mp 156-7°(d).

Example 13

2-(1H-2-Benzimidazolylsulphinylmethyl)-N-cyclohexyl-N-methyl-benzenamine

a) 2-(N-Cyclohexyl-N-methyl-amino)benzaldehyde

o-Fluorobenzaldehyde (8.68g) and N-methylcyclohexylamine (11.9g) were heated under reflux in dimethyl formamide (70ml) containing potassium carbonate (14.49g) with stirring for 5.5 hours. The cooled reaction mixture was poured into dilute HCl and extracted into $CHCl_3$. The aqueous layer was separated and basified with potassium carbonate and extracted into $CHCl_3$, which was then washed with water, dried and evaporated, to afford the sub-title compound (11.8g). MS:$M^+$ 217 BP 174.

b) 2-(N-Cyclohexyl-N-methylamino)benzene methanol

The product of step a) was reduced by the method of Example 5d) to afford the sub-title compound. MS:$M^+$ 219 BP 148.

c) 2-(1H-2-Benzimidazolylthiomethyl)-N-cyclohexyl-N-methyl-benzenamine

The product of step b) was converted to the sub-title compound by the method of Example 5, mp 165-166°.

d) 2-(1H-2-Benzimidazolylsulphinylmethyl)-N-cyclohexyl-N-methyl-benzenamine

The product of step c) was converted to the title compound by the method of Example 1b), mp 132-133°.

**Claims**

**Claims for the following Contracting States: DE, NL, SE**

**1.** A compound of formula I,

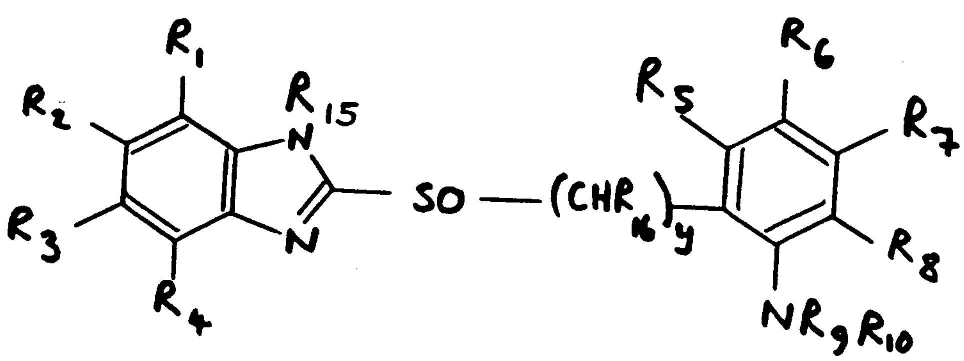

I

wherein $R_1$, $R_2$, $R_3$, $R_4$, $R_5$, $R_6$, $R_7$ and $R_8$, which may be the same or different, are each hydrogen, halogen, $C_{1-6}$-alkoxy, $C_{1-6}$-alkyl, $C_{1-6}$-fluoroalkyl, $RS(O)_n$-, or $-NO_2$; in addition, $R_2$ and $R_3$ may together form the chain -CH=CH-CH=CH-, and $R_1$, $R_2$, $R_3$ and $R_4$ may independently represent phenylcarbonyl;

$R_9$ and $R_{10}$, which may be the same or different, are each hydrogen, $C_{1-6}$-alkyl, phenyl or cycloalkyl containing up to 6 carbon atoms; or

$R_9$ and $R_{10}$, together with the nitrogen atom to which they are attached, may form a saturated or unsaturated 4 to 8 inclusive membered ring which may contain 0, 1 or 2 further hetero atoms, which ring may carry one or more substituents $R_1$, or

$R_9$ is as defined above save that it cannot form a ring with $R_{10}$, and $R_8$ and $R_{10}$, together with the nitrogen atom and the carbon atoms of the ring to which the nitrogen atom and $R_8$ are attached form a 4 to 8 inclusive membered ring which may contain 0, 1, or 2 further hetero atoms, which ring may carry one or more substituents $R_1$,

y is 0, 1 or 2;

n is 0, 1 or 2;

$R_{15}$ is hydrogen, -COOR or $C_{1-6}$-alkyl which latter is optionally substituted by -OCOR,

$R_{16}$ is H or $C_{1-6}$-alkyl

R is hydrogen, $C_{1-6}$-alkyl, phenyl, or phenyl substituted by $C_{1-6}$-alkyl;

and pharmaceutically acceptable salts thereof.

**2.** A compound of formula I defined in claim 1, wherein $R_9$ and $R_{10}$ each contain 1 or 2 carbon atoms.

**3.** A compound of formula I as defined in claim 1 or claim 2, wherein y is 1.

**4.** N,N-Dimethyl-2-(1H-benzimidazol-2-yl-sulphinylmethyl)benzenamine, or a pharmaceutically acceptable salt thereof.

**5.** N,N-Dimethyl-2-(5,6-dimethyl-1H-2-benzimidazolyl sulphinylmethyl)benzenamine,
2-(1H-2-Benzimidazolylsulphinylmethyl)-4-chloro-N,N-dimethyl-benzenamine,
2-(5,6-Dichloro-1H-2-benzimidazolylsulphinyl methyl)-N,N-dimethyl-benzenamine,
2-(1H-2-Benzimidazolylsulphinylmethyl)-N,N-diethyl-benzenamine,
N,N-Dimethyl-2-(5-nitro-1H-2-benzimidazolyl sulphinylmethyl)-benzenamine,
[2-(2-N,N-Dimethylaminophenylmethylsulphinyl)-1H-5-benzimidazolyl]phenyl methanone,
2-(5,6-Dimethoxy-1H-2-benzimidazolylsulphinyl methyl)-N,N-dimethyl-benzenamine,
N,N-Dimethyl-2-(4-trifluoromethyl-1H-2-benzimidazolylsulphinylmethyl)-benzenamine,
N,N-Dimethyl-2-(1H-2-naphtho[2,3-d]imidazolylsulphinylmethyl)-benzenamine,
2-[2-(1H-Benzimidazolyl)sulphinyl]-benzenamine
2-(1H-2-Benzimidazolylsulphinylmethyl)-N,N-dimethyl-6-propyl-benzenamine,
2-(1H-2-Benzimidazolylsulphinylmethyl)-N-ethyl-N-propyl-benzenamine,
2-(1,2,3,4-Tetrahydro-1,6-dimethylquinolin-8-ylmethylsulphinyl)-1H-benzimidazole,
2-(1H-2-Benzimidazolylsulphinylmethyl)-N,N,3,4,5-pentamethyl-benzenamine,
2-(1H-2-Benzimidazolylsulphinylmethyl)-4-methoxy-N,N,3,5-tetramethyl-benzenamine,
2-(1H-2-Benzimidazolylsulphinylmethyl)-N,N-dimethyl-4-(1,1-dimethylethyl)-benzenamine,
2-[1-(2-Dimethylaminophenyl)ethylsulphinyl]-1H-benzimidazole,
[2-(2-Dimethylaminophenylmethylsulphinyl)-1H-benzimidazol-1-yl]methyl-2,2-dimethylpropanoate,
Ethyl 2-(2-dimethylaminophenylmethylsulphinyl)-1H-1-benzimidazole carboxylate,
2-[1-Methyl-(1H-2-benzimidazolylsulphinylmethyl)]-N,N-dimethylbenzenamine,
N,N-Dimethyl-2-[5-(4-methylphenylsulphonyl)-1H-2-benzimidazolylsulphinylmethyl]-benzenamine,
2-(4,7-Dimethoxy-1H-2-benzimidazolylsulphinyl methyl)-N,N-dimethyl-benzenamine,
2-(1H-Benzimidazolylsulphinylmethyl)-N-cyclohexyl -N-methyl-benzenamine,
or a pharmaceutically acceptable salt of any one thereof.

**6.** A pharmaceutical formulation comprising a compound according to any one of the preceding claims in admixture with a pharmaceutically acceptable adjuvant, diluent or carrier.

**7.** A compound of formula I as defined in claim 1, or a pharmaceutically acceptable salt thereof, for use as a pharmaceutical.

**8.** A compound of formula I as defined in claim 1, for use in the manufacture of a medicament for use in the prevention or inhibition of gastric acid secretion.

**9.** A process for the production of a compound of formula I, as defined in Claim 1, or a pharmaceutically

acceptable salt thereof, which comprises selective oxidation of a corresponding compound of formula VI,

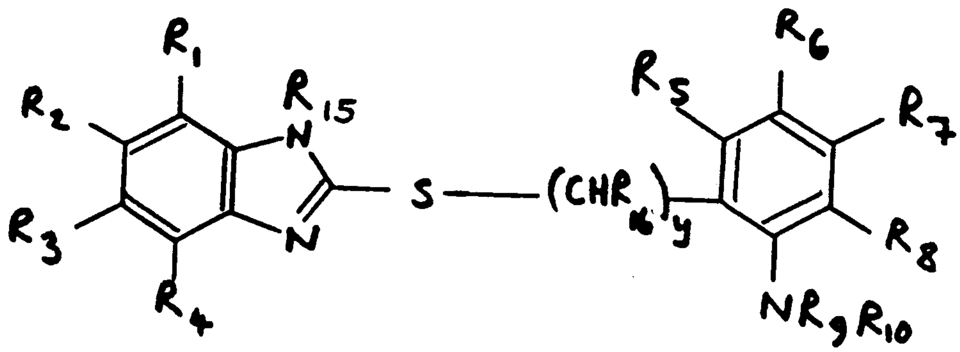

VI

in which $R_1$, $R_2$, $R_3$, $R_4$, $R_5$, $R_6$, $R_7$, $R_8$, $R_9$, $R_{10}$, $R_{15}$, $R_{16}$ and y are as defined in claim 1, and where desired or necessary converting the resulting compound of formula I to a pharmaceutically acceptable salt thereof, or vice versa.

**10.** A process for the production of a compound of formula I as defined in claim 1, in which $R_{15}$ is as defined in claim 1 save that it cannot be hydrogen, by reaction of a corresponding compound of formula I in which $R_{15}$ is hydrogen with a compound $R_{15}Z$ in which $R_{15}$ is as defined in Claim 1 save that it cannot be hydrogen, and Z is a good leaving group, and where desired or necessary converting the resulting compound of formula I to a pharmaceutically acceptable salt thereof, or vice versa.

**11.** A compound of formula VI as defined in claim 9.

**Claims for the following Contracting State: AT**

**1.** A process for the production of a compound of formula I,

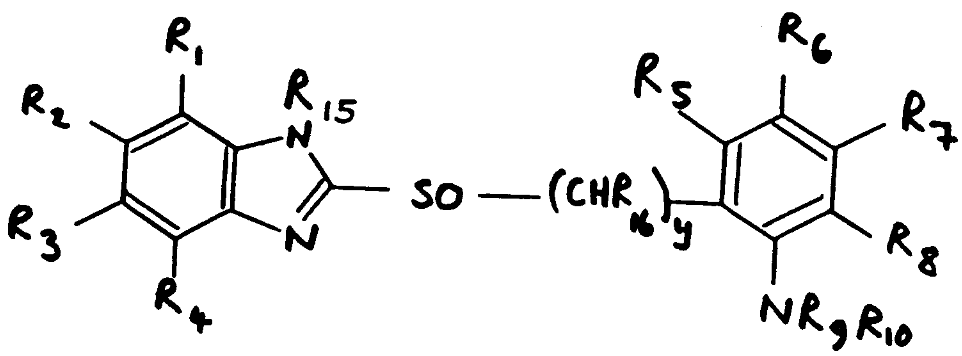

I

wherein $R_1$, $R_2$, $R_3$, $R_4$, $R_5$, $R_6$, $R_7$ and $R_8$, which may be the same or different, are each hydrogen, halogen, $C_{1-6}$-alkoxy, $C_{1-6}$-alkyl, $C_{1-6}$-fluoralkyl, $RS(O)_n$-, $-NO_2$, $-N(R)_2$, or -COOH or an ester or amide thereof,

in addition, $R_2$ and $R_3$ may together form the chain -CH=CH-CH=CH-, and $R_1$, $R_2$, $R_3$ and $R_4$ may independently represent phenylcarbonyl;

$R_9$ and $R_{10}$, which may be the same or different, are each hydrogen, $C_{1-6}$-alkyl, phenyl or cycloalkyl containing up to 6 carbon atoms; or

$R_9$ and $R_{10}$, together with the nitrogen atom to which they are attached may form a saturated or unsaturated 4 to 8 inclusive membered ring which may contain 0, 1 or 2 further hetero atoms, which ring may carry one or more substituents $R_1$, or

$R_9$ is as defined above save that it cannot form a ring with $R_{10}$, and $R_8$ and $R_{10}$, together with the nitrogen atom and the carbon atoms of the ring to which the nitrogen atom and $R_8$ are attached form a 4 to 8 inclusive membered ring which may contain 0, 1, or 2 further hetero atoms, which ring may carry

one or more substituents $R_1$,

y is 0, 1 or 2;

n is 0, 1 or 2;

$R_{15}$ is hydrogen, -COOR or $C_{1-6}$-alkyl which latter is optionally substituted by -OCOR,

$R_{16}$ is H or $C_{1-6}$-alkyl;

R is hydrogen, $C_{1-6}$-alkyl, phenyl, or phenyl substituted by $C_{1-6}$-alkyl;

or a pharmaceutically acceptable salt thereof;

which comprises selective oxidation of a corresponding compound of formula VI,

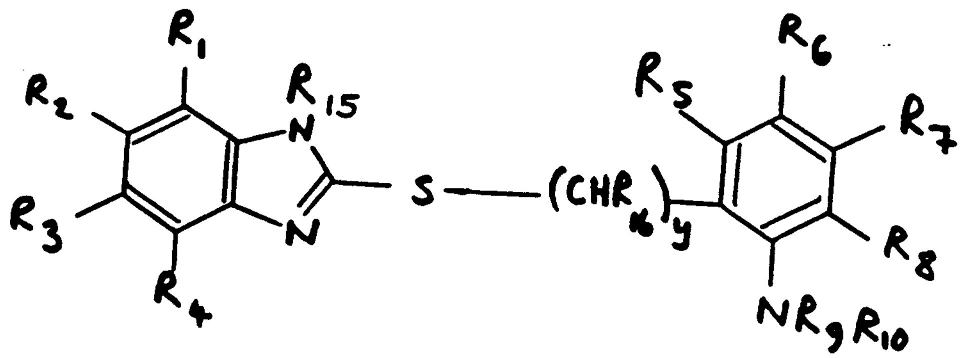

VI

in which $R_1$, $R_2$, $R_3$, $R_4$, $R_5$, $R_6$, $R_7$, $R_8$, $R_9$, $R_{15}$, $R_{16}$ and y are as defined above, and where desired or necessary converting the resulting compound of formula I to a pharmaceutically acceptable salt thereof, or vice versa.

**2.** A process for the production of a compound of formula I as defined in claim 1, wherein $R_9$ and $R_{10}$ each contain 1 or 2 carbon atoms.

**3.** A process for the production of a compound of formula I as defined in claim 1 or claim 2, wherein y is 1.

**4.** A process for the production of a compound of formula I as defined in any one of the preceding claims, wherein $R_{15}$ is as defined in claim 1 save that it cannot be hydrogen, by reaction of a corresponding compound of formula I in which $R_{15}$ is hydrogen with a compound $R_{15}Z$ in which $R_{15}$ is as defined above save that it cannot be hydrogen, and Z is a good leaving group, and where desired or necessary converting the resulting compound of formula I to a pharmaceutically acceptable salt thereof, or vice versa.

**5.** A process for the production of a compound of formula I as defined in any one of the preceding claims which carries an $-NH_2$ group, by selective reduction of a corresponding compound of formula I carrying an $-NO_2$ group, and where desired or necessary converting the resulting compound of formula I to a pharmaceutically acceptable salt thereof, or vice versa.

**6.** A process according to any one of the preceding claims, wherein $R_1$ to $R_4$ are selected from hydrogen, methoxycarbonyl, phenylcarbonyl, methyl, chloro, methoxy, $CF_3$, $NO_2$, p-toluenesulphonyl or $-NH_2$ or $R_2$ and $R_3$ together form the chain -CH = CH-CH = CH-.

**7.** A process according to any one of the preceeding claims, wherein,

$R_5$ to $R_8$ are selected from hydrogen, methyl, chloro, propyl, methoxyl or butyl, and $R_{15}$ is $-CH_2OCO-(^t butyl)$, $-CO_2Et$ or methyl.

**8.** A process according to claim 1, wherein the compound of formula I is N,N-Dimethyl-2-(1H-benzimidazol-2-ylsulphinylmethyl)-benzenamine, or a pharmaceutically acceptable salt thereof.

**9.** A process according to claim 1, wherein the compound of formula I is

N,N-Dimethyl-2-(5,6-dimethyl-1H-2-benzimidazolyl sulphinylmethyl)benzenamine,

2-(1H-2-Benzimidazolylsulphinylmethyl)-N,N,4-trimethyl-benzenamine,

2-(1H-2-Benzimidazolylsulphinlymethyl)-4-chloro-N,N-dimethyl-benzenamine,
2-(5-Chloro-1H-2-benzimidazolylsulphinylmethyl)-N,N-dimethyl-benzenamine,
2-(5,6-Dichloro-1H-2-benzimidazolylsulphinyl methyl)-N,N-dimethyl-benzenamine,
Methyl 2-(2-dimethylaminophenylmethylsulphinyl)-1H-benzimidazole-5-carboxylate,
N,N-Dimethyl-2-(5-methyl-1H-2-benzimidazolyl sulphinylmethyl)benzenamine,
2-[2-(1-Piperidyl)-phenylmethylsulphinyl]-1H-benzimidazole,
2-(1H-2-Benzimidazolylsulphinylmethyl)-N,N-diethyl-benzenamine,
2-[2-(5-Methoxy-1H-benzimidazolyl)sulphinyl methyl]-N,N-dimethyl-benzenamine,
2-(5-Trifluoromethyl-1H-2-benzimidazolylsulphinylmethyl)-N,N-dimethyl-benzenamine,
N,N-Dimethyl-2-(5-nitro-1H-2-benzimidazolyl sulphinylmethyl)-benzenamine,
[2-(2-N,N-Dimethylaminophenylmethylsulphinyl)-1H-5-benzimidazolyl]phenyl methanone,
2-(5,6-Dimethoxy-1H-2-benzimidazolylsulphinyl methyl)-N,N-dimethyl-benzenamine,
N,N-Dimethyl-2-(4-trifluoromethyl-1H-2-benzimidazolylsulphinylmethyl)-benzenamine,
N,N-Dimethyl-2-(1H-2-naphtho[2,3-d]imidazolylsulphinylmethyl)-benzenamine,
2-(2-(1H-Benzimidazolyl)sulphinyl)benzenamine
2-(1H-2-Benzimidazolylsulphinylmethyl)-N,N-dimethyl-6-propyl-benzenamine,
2-(1H-2-Benzimidazolylsulphinylmethyl)-4-methoxy-N,N-dimethyl-benzenamine,
2-(1H-2-Benzimidazolylsulphinylmethyl)-N-ethyl-N-propyl-benzenamine,
2-[2-(4-Morpholinyl)phenylmethylsulphinyl]-1H-benzimidazole,
2-(1,2,3,4-Tetrahydro-1,6-dimethylquinolin-8-ylmethylsulphinyl)-1H-benzimidazole,
2-(1H-2-Benzimidazolylsulphinylmethyl)-N,N,3,4,5-pentamethyl-benzeneamine,
2-(1H-2-Benzimidazolylsulphinylmethyl)-4-methoxy-N,N,3,5-tetramethyl-benzenamine,
2-(1H-2-Benzimidazolylsulphinylmethyl)-N,N-dimethyl-4-(1,1-dimethylethyl)-benzenamine,
2-[1-(2-Dimethylaminophenyl)ethylsulphinyl]-1H-benzimidazole,
[2-(2-Dimethylaminophenylmethylsulphinyl)-1H-benzimidazol-1-yl]methyl-2,2-dimethylpropanoate,
Ethyl 2-(2-dimethylaminophenylmethylsulphinyl)-1H-1-benzimidazole carboxylate,
2-[1-Methyl-(1H-2-benzimidazolylsulphinylmethyl)]-N,N-dimethylbenzenamine,
N,N-Dimethyl-2-[5-(4-methylphenylsulphonyl)-1H-2-benzimidazolylsulphinylmethyl]-benzenamine,
2-(4,7-Dimethoxy-1H-2-benzimidazolylsulphinyl methyl)-N,N-dimethyl-benzenamine,
2-(1H-2-Benzimidazolylsulphinylmethyl)benzenamine,
2-(5-Amino-1H-2-benzimidazolylsulphinylmethyl)-N,N-dimethyl-benzenamine,
2-(1H-2-Benzimidazolylsulphinylmethyl)-N-cyclohexyl-N-methyl-benzenamine,
or a pharmaceutically acceptable salt thereof.

**10.** A compound of formula I as defined in claim 1 for use in the manufacture of a medicament for use in the prevention or inhibition of gastric acid secretion.

**Revendications**

**Revendications pour les Etats contractants suivants: DE, NL SE**

**1.** Composé de formule I,

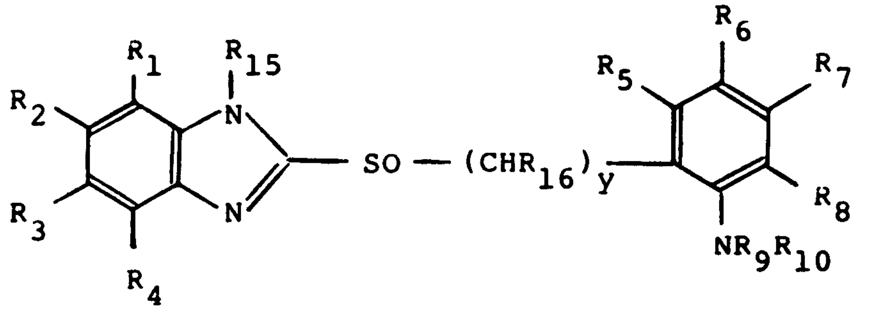

I

où $R_1$, $R_2$, $R_3$, $R_4$, $R_5$, $R_6$, $R_7$ et $R_8$, qui peuvent être identiques ou différents, sont chacun hydrogène, halogène, $C_{1-6}$-alcoxy, $C_{1-6}$-alcoyle, $C_{1-6}$-fluoroalcoyle, $RS(O)_n$- ou $-NO_2$;

en outre, $R_2$ et $R_3$ peuvent former ensemble la chaîne -CH=CH-CH=CH-, et $R_1$, $R_2$, $R_3$ et $R_4$ peuvent représenter indépendamment phénylcarbonyle;

$R_9$ et $R_{10}$, qui peuvent être identiques ou différents, sont chacun hydrogène, $c_{1-6}$-alcoyle, phényle ou cycloalcoyle comptant jusqu'à 6 atomes de carbone; ou bien

$R_9$ et $R_{10}$, conjointement avec l'atome d'azote auquel ils sont unis, peuvent former un cycle saturé ou insaturé de 4 à 8 chaînons inclusivement qui peut comprendre 0, 1 ou 2 hétéroatomes supplémentaires, lequel cycle peut porter un ou plusieurs substituants $R_1$, ou bien

$R_9$ est tel que défini ci-dessus, sauf qu'il ne peut former un cycle avec $R_{10}$, et $R_8$ et $R_{10}$, conjointement avec l'atome d'azote et les atomes de carbone du cycle auxquels l'atome d'azote et $R_8$ sont unis forment un cycle de 4 à 8 chaînons inclusivement qui peut contenir 0, 1 ou 2 hétéroatomes supplémentaires, lequel cycle peut porter un ou plusieurs substituants $R_1$,

y est 0, 1 ou 2;

n est 0, 1 ou 2;

$R_{15}$ est hydrogène, -COOR ou $C_{1-6}$-alcoyle, lequel dernier est éventuellement substitué par -OCOR;

$R_{16}$ est hydrogène ou $C_{1-6}$-alcoyle;

R est hydrogène, $C_{1-6}$-alcoyle, phényle ou phényle substitué par $C_{1-6}$-alcoyle;

et les sels pharmaceutiquement acceptables de celui-ci.

**2.** Composé de formule I tel que défini dans la revendication 1, où $R_9$ et $R_{10}$ comptent chacun 1 ou 2 atomes de carbone.

**3.** Composé de formule I tel que défini dans la revendication 1 ou 2, où y est 1.

**4.** La N,N-diméthyl-2-(1H-benzimidazol-2-yl-sulfinylméthyl)benzèneamine, ou un sel pharmaceutiquement acceptable de celle-ci.

**5.** La N,N-diméthyl-2-(5,6-diméthyl-1H-2-benzimidazolylsulfinylméthyl)benzèneamine,
la 2-(1H-2-benzimidazolylsulfinylméthyl)-4-chloro-N,N-diméthyl-benzèneamine,
la 2-(5,6-dichloro-1H-2-benzimidazolylsulfinylméthyl)-N,N-diméthyl-benzèneamine,
la 2-(1H-2-benzimidazolylsulfinylméthyl)-N,N-diéthyl-benzèneamine,
la N,N-diméthyl-2-(5-nitro-1H-2-benzimidazolylsulfinylméthyl)-benzèneamine,
la [2-(2-N,N-diméthylaminophénylméthylsulfinyl)-1H-5-benzimidazolyl]phényl-méthanone,
la 2-(5,6-diméthoxy-1H-2-benzimidazolylsulfinylméthyl)-N,N-diméthyl-benzèneamine,
la N,N-diméthyl-2-(4-trifluorométhyl-1H-2-benzimidazolylsulfinylméthyl)-benzèneamine,
la N,N-diméthyl-2-(1H-2-naphto[2,3-d]imidazolylsulfinylméthyl)-benzèneamine,
la 2-(2-1H-benzimidazolyl)sulfinyl)benzèneamine,
la 2-(1H-2-benzimidazolylsulfinylméthyl)-N,N-diméthyl-6-propyl-benzèneamine,
la 2-(1H-2-benzimidazolylsulfinylméthyl)-N-éthyl-N-propyl-benzèneamine,
le 2-(1,2,3,4-tétrahydro-1,6-diméthylquinoléin-8-ylméthylsulfinyl)-1H-benzimidazole,
la 2-(1H-2-benzimidazolylsulfinylméthyl)-N,N,3,4,5-pentaméthyl-benzèneamine,
la 2-(1H-2-benzimidazolylsulfinylméthyl)-4-méthoxy-N,N,3,5-tétraméthyl-benzèneamine,
la 2-(1H-2-benzimidazolylsulfinylméthyl)-N,N-diméthyl-4-(1,1-diméthyléthyl)-benzèneamine,
le 2-[1-(2-diméthylaminophényl)éthylsulfinyl]-1H-benzimidazole,
le 2,2-diméthylpropanoate de [2-(2-diméthylaminophénylméthylsulfinyl)-1H-benzimidazol-1-yl]-méthyle,
le 2-(2-diméthylaminophénylméthylsulfinyl)-1H-1-benzimidazole carboxylate d'éthyle,
la 2-[1-méthyl-(1H-2-benzimidazolylsulfinylméthyl)]-N,N-diméthylbenzèneamine,
la N,N-diméthyl-2-[5-(4-méthylphénylsulfonyl)-1H-2-benzimidazolylsulfinylméthyl]benzèneamine,
la 2-(4,7-diméthoxy-1H-2-benzimidazolylsulfinylméthyl)-N,N-diméthyl-benzèneamine,
la 2-(1H-2-benzimidazolylsulfinylméthyl)-N-cyclohexyl-N-méthyl-benzèneamine,
ou un sel pharmaceutiquement acceptable de l'un quelconque de ceux-ci.

**6.** Composition pharmaceutique comprenant un composé suivant l'une quelconque des revendications précédentes en mélange avec un adjuvant, diluant ou excipient pharmaceutiquement acceptable.

**7.** Composé de formule I tel que défini dans la revendication 1 ou un sel pharmaceutiquement acceptable de celui-ci, à utiliser comme agent pharmaceutique.

**8.** Composé de formule I tel que défini dans la revendication 1, à utiliser pour la fabrication d'un

médicament pour la prévention ou l'inhibition de la sécrétion d'acide gastrique.

**9.** Procédé de préparation d'un composé de formule I tel que défini dans la revendication 1 ou d'un sel pharmaceutiquement acceptable de celui-ci, qui comprend l'oxydation sélective d'un composé correspondant de formule VI,

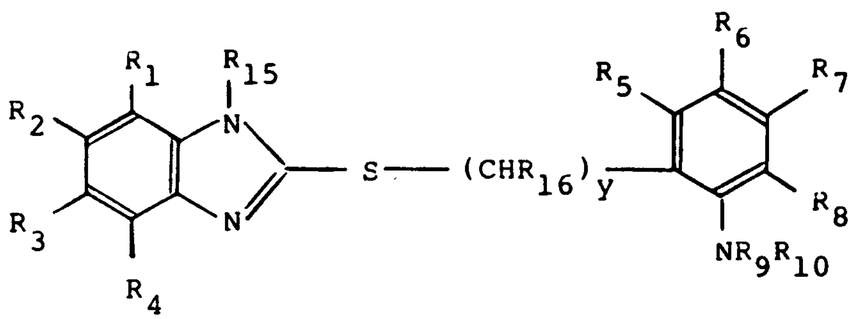

VI

où $R_1$, $R_2$, $R_3$, $R_4$, $R_5$, $R_6$, $R_7$, $R_8$, $R_9$, $R_{10}$, $R_{15}$, $R_{16}$ et y sont tels que définis dans la revendication 1, et lorsque la chose est souhaitée ou nécessaire, la conversion du composé résultant de formule I en un sel pharmaceutiquement acceptable de celui-ci, et vice versa.

**10.** Procédé de préparation d'un composé de formule I tel que défini dans la revendication 1, où $R_{15}$ est tel que défini dans la revendication 1, sauf qu'il ne peut être hydrogène, par réaction d'un composé correspondant de formule I, où $R_{15}$ est hydrogène, avec un composé $R_{15}Z$, où $R_{15}$ est tel que défini dans la revendication 1, sauf qu'il ne peut être hydrogène, et Z est un bon radical partant, et lorsque la chose est souhaitée ou nécessaire, par conversion du composé résultant de formule I en un sel pharmaceutiquement acceptable de celui-ci, et vice versa.

**11.** Composé de formule VI, tel que défini dans la revendication 9.

**Revendications pour l'Etat contractant suivant: AT**

**1.** Procédé de préparation d'un composé de formule I,

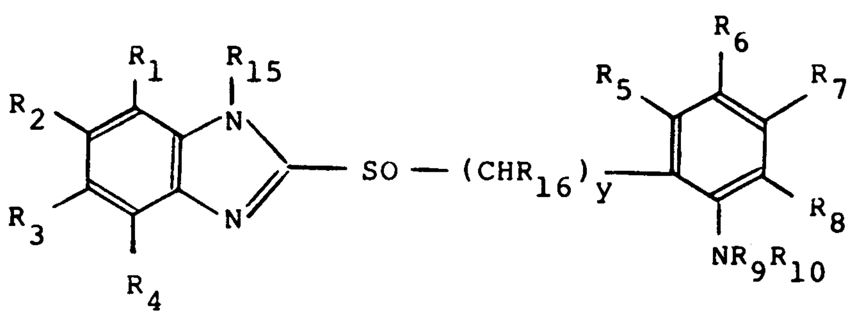

I

où $R_1$, $R_2$, $R_3$, $R_4$, $R_5$, $R_6$, $R_7$ et $R_8$, qui peuvent être identiques ou différents, sont chacun hydrogène, halogène, $C_{1-6}$-alcoxy, $C_{1-6}$-alcoyle, $C_{1-6}$-fluoroalcoyle, $RS(O)_n$-, $-NO_2$, $-N(R)_2$ ou -COOH ou un ester ou amide de celui-ci,

en outre, $R_2$ et $R_3$ peuvent former ensemble la chaîne -CH=CH-CH=CH-, et $R_1$, $R_2$, $R_3$ et $R_4$ peuvent représenter indépendamment phénylcarbonyle;

$R_9$ et $R_{10}$, qui peuvent être identiques ou différents, sont chacun hydrogène, $C_{1-6}$-alcoyle, phényle ou cycloalcoyle comptant jusqu'à 6 atomes de carbone; ou bien

$R_9$ et $R_{10}$, conjointement avec l'atome d'azote auquel ils sont unis, peuvent former un cycle saturé ou insaturé de 4 à 8 chaînons inclusivement qui peut comprendre 0, 1 ou 2 hétéroatomes supplémentaires, lequel cycle peut porter un ou plusieurs substituants $R_1$, ou bien

$R_9$ est tel que défini ci-dessus, sauf qu'il ne peut former un cycle avec $R_{10}$, et $R_8$ et $R_{10}$,

conjointement avec l'atome d'azote et les atomes de carbone du cycle auxquels l'atome d'azote et $R_8$ sont unis forment un cycle de 4 à 8 chaînons inclusivement qui peut contenir 0, 1 ou 2 hétéroatomes supplémentaires, lequel cycle peut porter un ou plusieurs substituants $R_1$,

y est 0, 1 ou 2;

n est 0, 1 ou 2;

$R_{15}$ est hydrogène, -COOR ou $C_{1-6}$-alcoyle, lequel dernier est éventuellement substitué par -OCOR;

$R_{16}$ est hydrogène ou $C_{1-6}$-alcoyle;

R est hydrogène, $C_{1-6}$-alcoyle, phényle ou phényle substitué par $C_{1-6}$-alcoyle;

ou d'un sel pharmaceutiquement acceptable de celui-ci; qui comprend l'oxydation sélective d'un composé correspondant de formule VI,

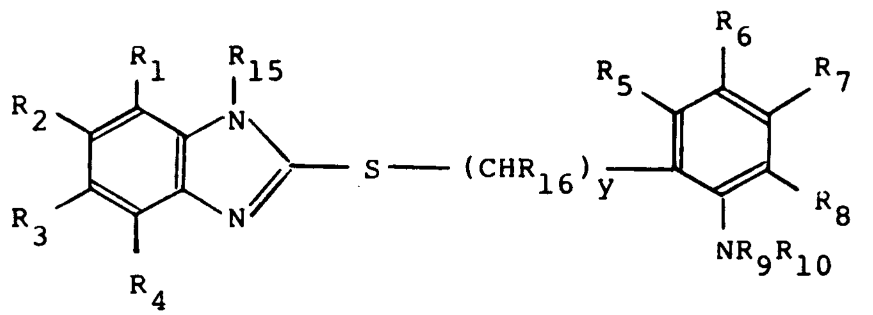

VI

où $R_1$, $R_2$, $R_3$, $R_4$, $R_5$, $R_6$, $R_7$, $R_8$, $R_9$, $R_{15}$, $R_{16}$ et y sont tels que définis ci-dessus, et lorsque la chose est souhaitée ou nécessaire, la conversion du composé résultant de formule I en un sel pharmaceutiquement acceptable de celui-ci et vice versa.

**2.** Procédé de préparation d'un composé de formule I tel que défini dans la revendication 1, où $R_9$ et $R_{10}$ comptent chacun 1 ou 2 atomes de carbone.

**3.** Procédé de préparation d'un composé de formule I tel que défini dans la revendication 1 ou 2, où y est 1.

**4.** Procédé de préparation d'un composé de formule I tel que défini dans l'une quelconque des revendications précédentes, où $R_{15}$ est tel que défini dans la revendication 1, sauf qu'il ne peut être hydrogène, par réaction d'un composé correspondant de formule I, où $R_{15}$ est hydrogène, avec un composé $R_{15}Z$ où $R_{15}$ est tel que défini ci-dessus sauf qu'il ne peut être hydrogène et Z est un bon radical partant, et lorsque la chose est souhaitée ou nécessaire, par conversion du composé résultant de formule I en un sel pharmaceutiquement acceptable de celui-ci et vice versa.

**5.** Procédé de préparation d'un composé de formule I tel que défini dans l'une quelconque des revendications précédentes qui porte un radical $-NH_2$, par réduction sélective d'un composé correspondant de formule I qui porte un radical $-NO_2$ et lorsque la chose est souhaitée ou nécessaire, par conversion du composé résultant de formule I en un sel pharmaceutiquement acceptable de celui-ci et vice versa.

**6.** Procédé suivant l'une quelconque des revendications précédentes, dans lequel $R_1$ à $R_4$ sont choisis parmi hydrogène, méthoxycarbonyle, phénylcarbonyle, méthyle, chloro, méthoxy, $CF_3$, $NO_2$, p-toluènesulfonyle et $-NH_2$, ou bien $R_2$ et $R_3$ forment ensemble la chaîne -CH=CH-CH=CH-.

**7.** Procédé suivant l'une quelconque des revendications précédentes, dans lequel $R_5$ à $R_8$ sont choisis parmi hydrogène, méthyle, chloro, propyle, méthoxy et butyle, et $R_{15}$ est $-CH_2OCO-(^t\text{butyle})$, $-CO_2Et$ ou méthyle.

**8.** Procédé suivant la revendication 1, dans lequel le composé de formule I est la N,N-diméthyl-2-(1H-benzimidazol-2-ylsulfinylméthyl)benzèneamine, ou un sel pharmaceutiquement acceptable de celle-ci.

9. Procédé suivant la revendication 1, dans lequel le composé de formule I est
la N,N-diméthyl-2-(5,6-diméthyl-1H-2-benzimidazolylsulfinylméthyl)benzèneamine,
la 2-(1H-2-benzimidazolylsulfinylméthyl)-N,N,4-triméthyl-benzèneamine,
la 2-(1H-2-benzimidazolylsulfinylméthyl)-4-chloro-N,N-diméthyl-benzèneamine,
la 2-(5-chloro-1H-2-benzimidazolylsulfinylméthyl)-N,N-diméthyl-benzèneamine,
la 2-(5,6-dichloro-1H-2-benzimidazolylsulfinylméthyl)-N,N-diméthyl-benzèneamine,
le 2-(2-diméthylaminophénylméthylsulfinyl)-1H-benzimidazole-5-carboxylate de méthyle,
la N,N-diméthyl-2-(5-méthyl-1H-2-benzimidazolylsulfinylméthyl)benzèneamine,
le 2-[2-(1-pipéridyl)-phénylméthylsulfinyl]-1H-benzimidazole,
la 2-(1H-2-benzimidazolylsulfinylméthyl)-N,N-diéthyl-benzèneamine,
la 2-[2-(5-méthoxy-1H-benzimidazolyl)sulfinylméthyl]-N,N-diméthyl-benzèneamine,
la 2-(5-trifluorométhyl-1H-2-benzimidazolylsulfinylméthyl)-N,N-diméthyl-benzèneamine,
la N,N-diméthyl-2-(5-nitro-1H-2-benzimidazolylsulfinylméthyl)-benzèneamine,
la [2-(2-N,N-diméthylaminophénylméthylsulfinyl)-1H-5-benzimidazolyl]phényl-méthanone,
la 2-(5,6-diméthoxy-1H-2-benzimidazolylsulfinylméthyl)-N,N-diméthyl-benzèneamine,
la N,N-diméthyl-2-(4-trifluorométhyl-1H-2-benzimidazolylsulfinylméthyl)-benzènamine,
la N,N-diméthyl-2-(1H-2-naphto[2,3-d]imidazolylsulfinylméthyl)-benzèneamine,
la 2-(2-1H-benzimidazolyl)sulfinyl)benzèneamine,
la 2-(1H-2-benzimidazolylsulfinylméthyl)-N,N-diméthyl-6-propyl-benzèneamine,
la 2-(1H-2-benzimidazolylsulfinylméthyl)-4-méthoxy-N,N-diméthyl-benzèneamine,
la 2-(1H-2-benzimidazolylsulfinylméthyl)-N-éthyl-N-propyl-benzèneamine,
le 2-[2-(4-morpholinyl)phénylméthylsulfinyl]-1H-benzimidazole,
le 2-(1,2,3,4-tétrahydro-1,6-diméthylquinoléin-8-ylméthylsulfinyl)-1H-benzimidazole,
la 2-(1H-2-benzimidazolylsulfinylméthyl)-N,N,3,4,5-pentaméthyl-benzèneamine,
la 2-(1H-2-benzimidazolylsulfinylméthyl)-4-méthoxy-N,N,3,5-tétraméthyl-benzèneamine,
la 2-(1H-2-benzimidazolylsulfinylméthyl)-N,N-diméthyl-4-(1,1-diméthyléthyl)-benzèneamine,
le 2-[1-(2-diméthylaminophényl)éthylsulfinyl]-1H-benzimidazole,
le 2,2-diméthylpropanoate de [2-(2-diméthylaminophénylméthylsulfinyl)-1H-benzimidazol-1-yl]-méthyle,
le 2-(2-diméthylaminophénylméthylsulfinyl)-1H-1-benzimidazole carboxylate d'éthyle,
la 2-[1-méthyl-(1H-2-benzimidazolylsulfinylméthyl)]-N,N-diméthylbenzèneamine,
la N,N-diméthyl-2-[5-(4-méthylphénylsulfonyl)-1H-2-benzimidazolylsulfinylméthyl]benzènamine,
la 2-(4,7-diméthoxy-1H-2-benzimidazolylsulfinylméthyl)-N,N-diméthyl-benzèneamine,
la 2-(1H-2-benzimidazolylsulfinylméthyl)benzèneamine,
la 2-(5-amino-1H-2-benzimidazolylsulfinylméthyl)-N,N-diméthyl-benzèneamine,
la 2-(1H-2-benzimidazolylsulfinylméthyl)-N-cyclohexyl-N-méthyl-benzèneamine,
ou un sel pharmaceutiquement acceptable de l'un quelconque de ceux-ci.

10. Composé de formule I tel que défini dans la revendication 1 à utiliser dans la fabrication d'un médicament pour la prévention ou l'inhibition de la sécrétion d'acide gastrique.

**Patentansprüche**
**Patentansprüche für folgende Vertragsstaaten: DE, NL, SE**

1. Verbindung der Formel (I)

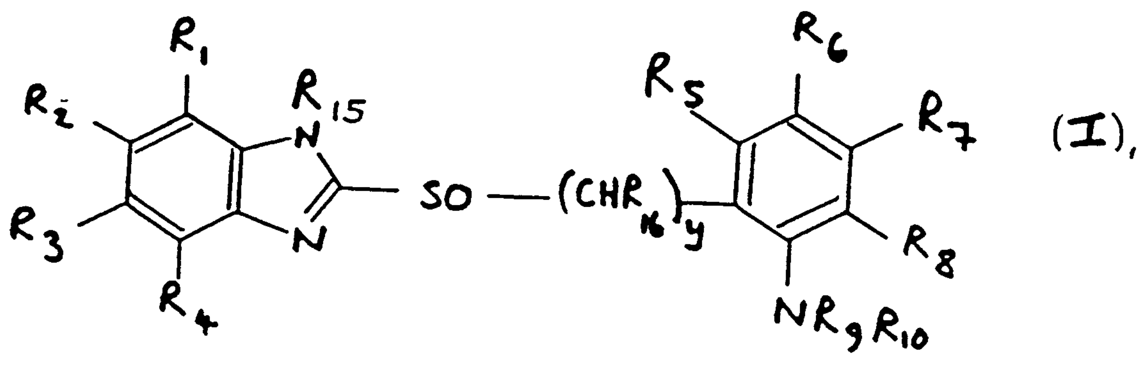

worin $R_1$, $R_2$, $R_3$, $R_4$, $R_5$, $R_6$, $R_7$ und $R_8$, die gleich oder verschieden sein können, jeweils Wasserstoff, Halogen, $C_1$-$C_6$-Alkoxy, $C_1$-$C_6$-Alkyl, $C_1$-$C_6$-Fluoralkyl, $RS(O)_n$- oder $-NO_2$ bedeuten,

$R_2$ und $R_3$ außerdem miteinander die Kette -CH = CH-CH = CH-bilden können und $R_1$, $R_2$, $R_3$ und $R_4$ unabhängig Phenylcarbonyl darstellen können,

$R_9$ und $R_{10}$, die gleich oder verschieden sein können, jeweils Wasserstoff, $C_1$-$C_6$-Alkyl, Phenyl oder Cycloalkyl mit bis zu 6 Kohlenstoffatomen bedeuten oder

$R_9$ und $R_{10}$ zusammen mit dem Stickstoffatom, an das sie gebunden sind, einen gesättigten oder ungesättigten 4- bis inkl. 8-gliedrigen Ring bilden können, der Null, 1 oder 2 weitere Heteroatome enthalten kann, welcher Ring einen oder mehrere Substituenten $R_1$ tragen kann, oder

$R_9$ wie oben definiert ist, vorausgesetzt, daß er mit $R_{10}$ keinen Ring bilden kann und $R_8$ und $R_{10}$ zusammen mit dem Stickstoffatom und den Kohlenstoffatomen des Ringes, an den das Stickstoffatom und $R_8$ gebunden sind, einen 4- bis inkl. 8-gliedrigen Ring bilden, der Null, 1 oder 2 weitere Heteroatome enthalten kann, welcher Ring einen oder mehrere Substituenten $R_1$ tragen kann,

y Null, 1 oder 2 ist,

n Null, 1 oder 2 ist,

$R_{15}$ Wasserstoff, -COOR oder $C_1$-$C_6$-Alkyl, welch letzteres gegebenenfalls durch -OCOR substituiert ist, darstellt,

$R_{16}$ H oder $C_1$-$C_6$-Alkyl bedeutet und

R Wasserstoff, $C_1$-$C_6$-Alkyl, Phenyl oder Phenyl substituiert durch $C_1$-$C_8$-Alkyl ist,

und pharmazeutisch annehmbare Salze hievon.

**2.** Verbindung der Formel (I), wie in Anspruch 1 definiert, worin $R_9$ und $R_{10}$ jeweils 1 oder 2 Kohlenstoffatome enthalten.

**3.** Verbindung der Formel (I), wie in Anspruch 1 oder 2 definiert, worin y 1 ist.

**4.** N,N-Dimethyl-2-(1H-benzimidazol-2-ylsulfinylmethyl)-benzolamin oder ein pharmazeutisch annehmbares Salz hievon.

**5.** N,N-Dimethyl-2-(5,6-dimethyl-1H-2-benzimidazolylsulfinylmethyl)-benzolamin,
2-(1H-2-Benzimidazolylsulfinylmethyl)-4-chlor-N,N-dimethylbenzolamin,
2-(5,6-Dichlor-1H-2-benzimidazolylsulfinylmethyl)-N,N-dimethylbenzolamin,
2-(1H-2-Benzimidazolylsulfinylmethyl)-N,N-diäthylbenzolamin, N,N-Dimethyl-2-(5-nitro-1H-2-benzimidazolylsulfinylmethyl)-benzolamin,
[2-(2-N,N-Dimethylaminophenylmethylsulfinyl)-1H-5-benzimidazolyl]-phenylmethanon,
2-(5,6-Dimethoxy-1H-2-benzimidazolylsulfinylmethyl)-N,N-dimethylbenzolamin,
N,N-Dimethyl-2-(4-trifluormethyl-1H-2-benzimidazolylsulfinylmethyl)-benzolamin,
N,N-Dimethyl-2-(1H-2-naphtho[2,3-d]imidazolylsulfinylmethyl)-benzolamin,
2-[2-(1H-Benzimidazolyl)-sulfinyl]-benzolamin,
2-(1H-2-Benzimidazolylsulfinylmethyl)-N,N-dimethyl-6-propylbenzolamin,
2-(1H-2-Benzimidazolylsulfinylmethyl)-N-äthyl-N-propylbenzolamin,
2-(1,2,3,4-Tetrahydro-1,6-dimethylchinolin-8-ylmethylsulfinyl)-1H-benzimidazol,
2-(1H-2-Benzimidazolylsulfinylmethyl)-N,N,3,4,5-pentamethylbenzolamin,
2-(1H-2-Benzimidazolylsulfinylmethyl)-4-methoxy-N,N,3,5-tetramethylbenzolamin,
2-(1H-2-Benzimidazolylsulfinylmethyl)-N,N-dimethyl-4-(1,1-dimethyläthyl)-benzolamin,
2-[1-(2-Dimethylaminophenyl)-äthylsulfinyl]-1H-benzimidazol,
[2-(2-Dimethylaminophenylmethylsulfinyl)-1H-benzimidazol-1-yl]-methyl-2,2-dimethylpropanoat,
Äthyl-2-(2-dimethylaminophenylmethylsulfinyl)-1H-1-benzimidazolcarboxylat,
2-[1-Methyl-(1H-2-benzimidazolylsulfinylmethyl)]-N,N-dimethylbenzolamin,
N,N-Dimethyl-2-[5-(4-methylphenylsulfonyl)-1H-2-benzimidazolylsulfinylmethyl]benzolamin,
2-(4,7-Dimethoxy-1H-2-benzimidazolylsulfinylmethyl)-N,N-dimethylbenzolamin,
2-(1H-2-Benzimidazolylsulfinylmethyl)-N-cyclohexyl-N-methylbenzolamin
oder ein pharmazeutisch annehmbares Salz eines derselben.

**6.** Pharmazeutische Formulierung umfassend eine Verbindung nach einem der vorhergehenden Ansprüche in Mischung mit einem pharmazeutisch annehmbaren Adjuvans, Verdünnungsmittel oder Träger.

**7.** Verbindung der Formel (I), wie in Anspruch 1 definiert, oder ein pharmazeutisch annehmbares Salz

hievon zur Verwendung als Pharmazeutikum.

8. Verbindung der Formel (I), wie in Anspruch 1 definiert, zur Verwendung bei der Herstellung eines Medikaments zur Verwendung bei der Verhütung oder Inhibierung von Magensäuresekretion.

9. Verfahren zur Herstellung einer Verbindung der Formel (I), wie in Anspruch 1 definiert, oder eines pharmazeutisch annehmbaren Salzes hievon, das die selektive Oxidation einer entsprechenden Verbindung der Formel (VI)

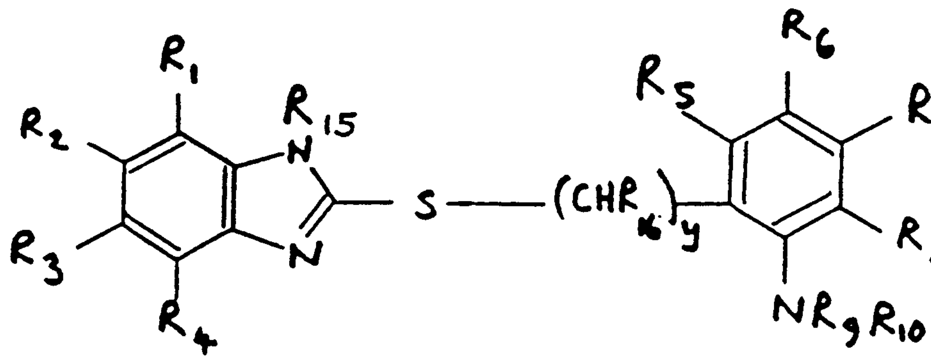

(VI),

worin $R_1$, $R_2$, $R_3$, $R_4$, $R_5$, $R_6$, $R_7$, $R_8$, $R_9$, $R_{10}$, $R_{15}$, $R_{16}$ und y wie in Anspruch 1 definiert sind, und, wenn erwünscht oder notwendig, das Überführen der erhaltenen Verbindung der Formel (I) in ein pharmazeutisch annehmbares Salz hievon oder vice versa umfaßt.

10. Verfahren zur Herstellung einer Verbindung der Formel (I), wie in Anspruch 1 definiert, worin $R_{15}$ wie in Anspruch 1 definiert ist, ausgenommen daß es nicht Wasserstoff sein kann, durch Umsetzen einer entsprechenden Verbindung der Formel (I), worin $R_{15}$ Wasserstoff ist, mit einer Verbindung $R_{15}Z$, worin $R_{15}$ wie in Anspruch 1 definiert ist, ausgenommen daß es nicht Wasserstoff sein kann, und Z eine gut abspaltbare Gruppe ist, und, wenn erwünscht oder notwendig, Überführen der erhaltenen Verbindung der Formel (I) in ein pharmazeutisch annehmbares Salz hievon oder vice versa.

11. Verbindung der Formel (VI), wie in Anspruch 9 definiert.

**Patentansprüche für folgenden Vertragsstaat: AT**

1. Verfahren zur Herstellung einer Verbindung der Formel (I)

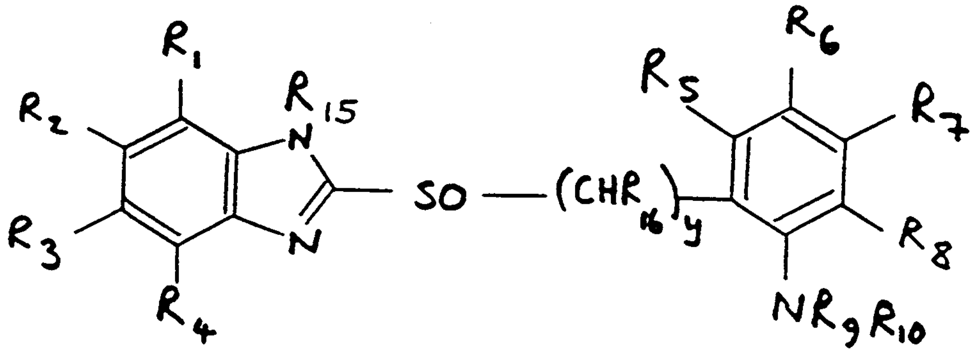

(I),

worin $R_1$, $R_2$, $R_3$, $R_4$, $R_5$, $R_6$, $R_7$ und $R_8$, die gleich oder verschieden sein können, jeweils Wasserstoff, Halogen, $C_1$-$C_6$-Alkoxy, $C_1$-$C_6$-Alkyl, $C_1$-$C_6$-Fluoralkyl, $RS(O)_n$-, $-NO_2$, $N(R)_2$ oder -COOH oder einen Ester oder ein Amid hievon bedeuten,

$R_2$ und $R_3$ außerdem miteinander die Kette -CH=CH-CH=CH- bilden können und $R_1$, $R_2$, $R_3$ und $R_4$ unabhängig Phenylcarbonyl darstellen können,

$R_9$ und $R_{10}$, die gleich oder verschieden sein können, jeweils Wasserstoff, $C_1$-$C_6$-Alkyl, Phenyl

oder Cycloalkyl mit bis zu 6 Kohlenstoffatomen bedeuten oder

$R_9$ und $R_{10}$ zusammen mit dem Stickstoffatom, an das sie gebunden sind, einen gesättigten oder ungesättigten 4- bis inkl. 8-gliedrigen Ring bilden können, der Null, 1 oder 2 weitere Heteroatome enthalten kann, welcher Ring einen oder mehrere Substituenten $R_1$ tragen kann, oder

$R_9$ wie oben definiert ist, vorausgesetzt, daß er mit $R_{10}$ keinen Ring bilden kann und $R_8$ und $R_{10}$ zusammen mit dem Stickstoffatom und den Kohlenstoffatomen des Ringes, an den das Stickstoffatom und $R_8$ gebunden sind, einen 4- bis inkl. 8-gliedrigen Ring bilden, der Null, 1 oder 2 weitere Heteroatome enthalten kann, welcher Ring einen oder mehrere Substituenten $R_1$ tragen kann,

y Null, 1 oder 2 ist,

n Null, 1 oder 2 ist,

$R_{15}$ Wasserstoff, -COOR oder $C_1$-$C_6$-Alkyl, welch letzteres gegebenenfalls durch -OCOR substituiert ist, darstellt,

$R_{16}$ Wasserstoff oder $C_1$-$C_6$-Alkyl bedeutet,

R Wasserstoff, $C_1$-$C_6$-Alkyl, Phenyl oder Phenyl substituiert durch $C_1$-$C_6$-Alkyl ist,

oder eines pharmazeutisch annehmbaren Salzes hievon, das die selektive Oxidation einer entsprechenden Verbindung der Formel (VI)

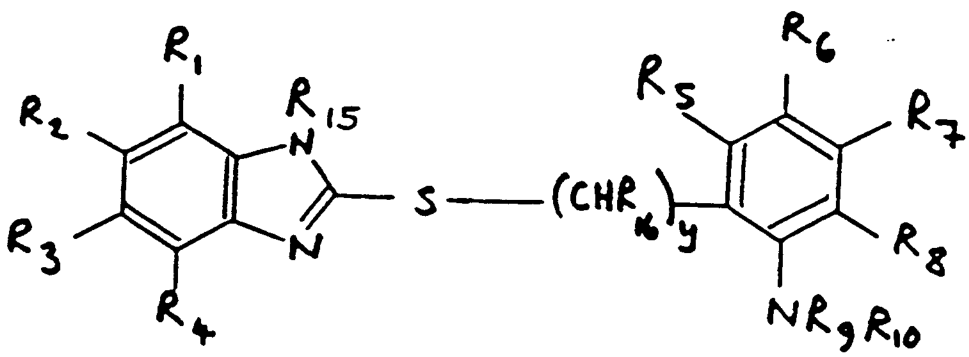

(VI),

worin $R_1$, $R_2$, $R_3$, $R_4$, $R_5$, $R_6$, $R_7$, $R_8$, $R_9$, $R_{10}$, $R_{15}$, $R_{16}$ und y wie oben definiert sind, und, wenn erwünscht oder notwendig, das Überführen der erhaltenen Verbindung der Formel (I) in ein pharmazeutisch annehmbares Salz hievon oder vice versa umfaßt.

**2.** Verfahren zur Herstellung einer Verbindung der Formel (I), wie in Anspruch 1 definiert, worin $R_9$ und $R_{10}$ jeweils 1 oder 2 Kohlenstoffatome enthalten.

**3.** Verfahren zur Herstellung einer Verbindung der Formel (I), wie in Anspruch 1 oder 2 definiert, worin y 1 ist.

**4.** Verfahren zur Herstellung einer Verbindung der Formel (I), wie in einem der vorhergehenden Ansprüche definiert, worin $R_{15}$ wie in Anspruch 1 definiert ist, ausgenommen daß es nicht Wasserstoff sein kann, durch Umsetzen einer entsprechenden Verbindung der Formel (I), worin $R_{15}$ Wasserstoff ist, mit einer Verbindung $R_{15}$ Z, worin $R_{15}$ wie oben definiert ist, ausgenommen daß es nicht Wasserstoff sein kann, und Z eine gut abspaltbare Gruppe ist, und, wenn erwünscht oder notwendig, Überführen der erhaltenen Verbindung der Formel (I) in ein pharmazeutisch annehmbares Salz hievon oder vice versa.

**5.** Verfahren zur Herstellung einer Verbindung der Formel (I), wie in einem der vorhergehenden Ansprüche definiert, die eine Gruppe $-NH_2$ trägt, durch selektive Reduktion einer entsprechenden Verbindung der Formel (I), die eine Gruppe $-NO_2$ trägt, und, wenn erwünscht oder notwendig, Überführen der erhaltenen Verbindung der Formel (I) in ein pharmazeutisch annehmbares Salz hievon oder vice versa.

**6.** Verfahren nach einem der vorhergehenden Ansprüche, worin $R_1$ bis $R_4$ ausgewählt sind aus Wasserstoff, Methoxycarbonyl, Phenylcarbonyl, Methyl, Chlor, Methoxy, $CF_3$, $NO_2$, p-Toluolsulfonyl oder $-NH_2$ oder $R_2$ und $R_3$ miteinander die Kette -CH = CH-CH = CH- bilden.

**7.** Verfahren nach einem der vorhergehenden Ansprüche, worin $R_5$ bis $R_8$ ausgewählt sind aus Wasserstoff, Methyl, Chlor, Propyl, Methoxy oder Butyl und $R_{15}$ $-CH_2OCO$-(tert.Butyl), $-CO_2Et$ oder Methyl

darstellt.

**8.** Verfahren nach Anspruch 1, worin die Verbindung der Formel (I) N,N-Dimethyl-2-(1H-benzimidazol-2-ylsulfinylmethyl)-benzolamin oder ein pharmazeutisch annehmbares Salz hievon ist.

**9.** Verfahren nach Anspruch 1, worin die Verbindung der Formel (I)
N,N-Dimethyl-2-(5,6-dimethyl-1H-2-benzimidazolylsulfinylmethyl)-benzolamin,
2-(1H-2-Benzimidazolylsulfinylmethyl)-N,N-4-trimethylbenzolamin,
2-(1H-2-Benzimidazolylsulfinylmethyl)-4-chlor-N,N-dimethylbenzolamin,
2-(5-Chlor-1H-2-benzimidazolylsulfinylmethyl)-N,N-dimethylbenzolamin,
2-(5,6-Dichlor-1H-2-benzimidazolylsulfinylmethyl)-N,N-dimethylbenzolamin,
Methyl-2-(2-dimethylaminophenylmethylsulfinyl)-1H-benzimidazol-5-carboxylat,
N,N-Dimethyl-2-(5-methyl-1H-2-benzimidazolylsulfinylmethyl)-benzolamin,
2-[2-(1-Piperidyl)-phenylmethylsulfinyl]-1H-benzimidazol,
2-(1H-2-Benzimidazolylsulfinylmethyl)-N,N-diäthylbenzolamin,
2-[2-(5-Methoxy-1H-benzimidazolyl)-sulfinylmethyl]-N,N-dimethylbenzolamin,
2-(5-Trifluormethyl-1H-2-benzimidazolylsulfinylmethyl)-N,N-dimethylbenzolamin,
N,N-Dimethyl-2-(5-nitro-1H-2-benzimidazolylsulfinylmethyl)-benzolamin,
[2-(2-N,N-Dimethylaminophenylmethylsulfinyl)-1H-5-benzimidazolyl]-phenylmethanon,
2-(5,6-Dimethoxy-1H-2-benzimidazolylsulfinylmethyl)-N,N-dimethylbenzolamin,
N,N-Dimethyl-2-(4-trifluormethyl-1H-2-benzimidazolylsulfinylmethyl)-benzolamin,
N,N-Dimethyl-2-(1H-2-naphtho[2,3-d]imidazolylsulfinylmethyl)-benzolamin,
2-[2-(1H-Benzimidazolyl)-sulfinyl]-benzolamin,
2-(1H-2-Benzimidazolylsulfinylmethyl)-N,N-dimethyl-6-propylbenzolamin,
2-(1H-2-Benzimidazolylsulfinylmethyl)-4-methoxy-N,N-dimethylbenzolamin,
2-(1H-2-Benzimidazolylsulfinylmethyl)-N-äthyl-N-propylbenzolamin,
2-[2-(4-Morpholinyl)-phenylmethylsulfinyl]-1H-benzimidazol,
2-(1,2,3,4-Tetrahydro-1,6-dimethylchinolin-8-ylmethylsulfinyl)-1H-benzimidazol,
2-(1H-2-Benzimidazolylsulfinylmethyl)-N,N,3,4,5-pentamethylbenzolamin,
2-(1H-2-Benzimidazolylsulfinylmethyl)-4-methoxy-N,N,3,5-tetramethylbenzolamin,
2-(1H-2-Benzimidazolylsulfinylmethyl)-N,N-dimethyl-4-(1,1-dimethyläthyl)-benzolamin,
2-[1-(2-Dimethylaminophenyl)-äthylsulfinyl]-1H-benzimidazol,
[2-(2-Dimethylaminophenylmethylsulfinyl)-1H-benzimidazol-1-yl]-methyl-2,2-dimethylpropanoat,
Äthyl-2-(2-dimethylaminophenylmethylsulfinyl)-1H-1-benzimidazolcarboxylat,
2-[1-Methyl-(1H-2-benzimidazolylsulfinylmethyl)]-N,N-dimethylbenzolamin,
N,N-Dimethyl-2-[5-(4-methylphenylsulfonyl)-1H-2-benzimidazolylsulfinylmethyl]-benzolamin,
2-(4,7-Dimethoxy-1H-2-benzimidazolylsulfinylmethyl)-N,N-dimethylbenzolamin,
2-(1H-2-Benzimidazolylsulfinylmethyl)-benzolamin,
2-(5-Amino-1H-2-benzimidazolylsulfinylmethyl)-N,N-dimethylbenzolamin,
2-(1H-2-Benzimidazolylsulfinylmethyl)-N-cyclohexyl-N-methylbenzolamin
oder ein pharmazeutisch annehmbares Salz hievon ist.

**10.** Verbindung der Formel (I), wie in Anspruch 1 definiert, zur Verwendung bei der Herstellung eines Medikaments zur Verwendung bei der Verhütung oder Inhibierung von Magensäuresekretion.